# EUROPÄISCHE PATENTSCHRIFT

(11) **EP 3 463 034 B1**
(45) Veröffentlichungstag und Bekanntmachung des Hinweises auf die Patenterteilung: **01.04.2020**
(21) Anmeldenummer: 17730064.7
(22) Anmeldetag: 23.05.2017
(51) Int. Cl.: A61B 1/00, A61B 46/10, H01R 13/52

(54) **ANORDNUNG ZUR STERILEN HANDHABUNG VON NICHT STERILEN EINHEITEN IN EINER STERILEN UMGEBUNG**
ARRANGEMENT FOR THE STERILE HANDLING OF NON-STERILE UNITS IN A STERILE ENVIRONMENT
DISPOSITION POUR LA MANIPULATION STÉRILE D'UNITÉS NON STÉRILES DANS UN ENVIRONNEMENT STÉRILE

(30) Priorität: 25.05.2016 DE 102016109601
(43) Veröffentlichungstag der Anmeldung: 10.04.2019
(73) Patentinhaber: avateramedical GmbH, 07745 Jena (DE)
(72) Erfinder: WEISE, Fabian, 10437 Berlin (DE); LÜCK, Martin, 73828 Esslingen (DE); VOGELSANG, Hartmut, 34399 Oberweser (DE); Preuß, Peter, 15537 Gosen-Neu Zittau (DE)
(74) Vertreter: Schaumburg und Partner Patentanwälte mbB
(86) Internationale Anmeldenummer: PCT/EP2017/062379
(87) Internationale Veröffentlichungsnummer: WO 2017/202831

(56) Entgegenhaltungen:
- DE-A1-102010 022 429
- DE-U1- 29 905 355
- US-A- 5 924 977
- US-A1- 2009 215 311

## Beschreibung

Die Erfindung betrifft eine erste Anordnung zur sterilen Handhabung eines nicht sterilen Endoskops in einer sterilen Umgebung. Die Anordnung umfasst eine sterile Endoskophülle zur Aufnahme des Endoskops, ein Kabel, über das das Endoskop mit einer Steuereinheit verbindbar ist und eine sterile Kabelhülle zur Aufnahme zumindest eines Abschnitts des Kabels. Ferner betrifft die Erfindung eine zweite Anordnung zum Herstellen und Lösen einer Steckverbindung zwischen einem ersten nicht sterilen Steckverbinder und einem zum ersten Steckverbinder komplementären zweiten nicht sterilen Steckverbinder in einer sterilen Umgebung. Diese zweite Anordnung umfasst eine erste sterile Hülle zur Aufnahme zumindest des ersten Steckverbinders und eine zweite sterile Hülle zur Aufnahme zumindest des zweiten Steckverbinders.

Der Erfindung liegt die Idee zugrunde, ein nicht steriles Endoskop in einem sterilen Umfeld, wie einem Operationssaal, zu verwenden, ohne das Endoskop sterilisieren zu müssen. Grundsätzlich wäre es zwar auch möglich, sterile Einmalendoskope zu verwenden, die nur für eine einzige Operation genutzt werden, jedoch ist dies weder aus ökonomischer Sicht noch aus Sicht des schonenden Umgangs mit Ressourcen sinnvoll. Zusammen mit dem Endoskop sollten auch Verbindungskabel zwischen einer Steuereinheit und dem Endoskop bei Mehrfachverwendung nicht vor jeder Verwendung sterilisiert werden müssen. Zur Verwendung von nicht sterilen Endoskopen in einem sterilen Umfeld ist aus dem Dokument DE 10 2010 022 429 A1 eine Hülle für ein Endoskop bekannt, die zwei Hüllenteile hat, die lösbar und fluiddicht mechanisch miteinander verbunden werden können. Ein Verbindungskabel zum Endoskop ist bei dieser Ausführungsform fest mit dem Endoskop verbunden, wobei nicht vorgesehen ist, dass sich in einem Schutzschlauch befindliche Kabel im sterilen Umfeld vom Endoskop zu trennen.

Aus dem Dokument WO 2012/075989 A1 ist ein handbetätigtes Endoskop für medizinische Zwecke bekannt, die eine separate modulartige Baueinheit und ein Übertragungssystem umfasst. Die Baueinheit ist nicht steril und kann in ein sterilisiertes Gehäuse eingesetzt werden.

Aus dem Dokument DE 10 2012 008 535 A1 ist ein Chirurgierobotersystem bekannt, bei dem ein steriles Endoskop mit einem unsterilen Manipulatorarm verbunden wird. Hierzu ist ein steriler Instrumentenadapter zwischen dem Manipulatorarm und dem Endoskop vorgesehen.

Nachteilig an diesen bekannten Lösungen ist, dass das Endoskop im sterilen Bereich nicht von dem Verbindungskabel trennbar und gegen ein anderes Endoskop austauschbar ist, ohne die Sterilität im sterilen Umfeld zu gefährden. Damit ist in der Praxis kein Wechseln des Endoskops im sterilen Bereich während einer Operation möglich. Sollte ein Endoskop während einer Operation einen Defekt aufweisen und ersetzt werden müssen, oder sollte während einer Operation ein eingesetztes Endoskop gegen ein weiteres Endoskop mit anderen optischen und/oder geometrischen Eigenschaften ausgetauscht werden, so müsste dieses zweite Endoskop zusammen mit einem sterilen Kabel bereits vor der Operation steril verpackt werden und dann während der Operation gegen das zweite steril verpackte Endoskop ausgetauscht werden. Alternativ können sterile Endoskope und/oder sterile Kabel auch im sterilen Umfeld gegeneinander ausgetauscht werden. Jedoch ist es sehr aufwändig, Endoskope und Kabel vor jeder Verwendung zu sterilisieren. Hierzu ist es erforderlich, dass das Endoskop nach jeder Verwendung gereinigt und autoklaviert wird. Im Autoklaven ist das Endoskop Wasserdampf bei einer Temperatur von ca. 140 °C und einem Druck von mehreren bar ausgesetzt, so dass an das Endoskop erhebliche thermische und mechanische Anforderungen gestellt werden müssen, damit dieses den Sterilisationsprozess im Autoklaven sicher übersteht.

Ähnliche Probleme treten bei der Verbindung mehrerer Steckverbinder auf, die entweder direkt an steril verpackten Geräten und/oder an Kabelenden vorgesehen sind.

Aufgabe der Erfindung ist es, eine Anordnung zur sterilen Handhabung eines nicht sterilen Endoskops in einer sterilen Umgebung sowie eine Anordnung zum Herstellen und Lösen einer Steckverbindung zwischen einem ersten nicht sterilen Steckverbinder und einem zweiten nicht sterilen Steckverbinder in einer sterilen Umgebung anzugeben, die das Herstellen und Trennen einer Verbindung zwischen dem Endoskop und einem Kabel bzw. zwischen zwei Steckverbindern ermöglichen, ohne die sterile Umgebung zu kontaminieren. Darüber hinaus ist ein System zur robotergestützten Chirurgie, insbesondere zu einer telerobotergestützten Prozedur innerhalb eines sterilen Bereichs anzugeben, bei der das Endoskop im sterilen Bereich mit Hilfe eines Manipulatorarms eines Manipulators positionierbar ist.

Diese Aufgabe wird durch eine Anordnung zur sterilen Handhabung eines nicht sterilen Endoskops in einer sterilen Umgebung mit den Merkmalen des Anspruchs 1, durch eine Anordnung zur robotergestützten Chirurgie mit den Merkmalen des Anspruchs 14 sowie durch eine Anordnung zum Herstellen und Lösen einer Steckverbindung zwischen einem ersten sterilen Steckverbinder und einem zweiten sterilen Steckverbinder in einer sterilen Umgebung mit den Merkmalen des Anspruchs 15 gelöst. Vorteilhafte Weiterbildungen sind in den abhängigen Ansprüchen angegeben.

Durch eine Anordnung zur sterilen Handhabung eines nicht sterilen Endoskops in einer sterilen Umgebung mit den Merkmalen des Anspruchs 1 wird erreicht, dass eine direkte Verbindung zwischen dem nicht sterilen Endoskop und mit dem nicht sterilen Kabel bzw. mit dem nicht sterilen mit dem Kabel verbundenen Steckverbinder sowohl sicher herstellbar als auch trennbar ist, ohne dass die Gefahr besteht, dass der sterile Bereich kontaminiert werden kann. Dies wird insbesondere durch die erste Sterilschleuse mit der ersten Sterilklappe und die zweite Sterilschleuse mit der zweiten Sterilklappe erreicht. Dadurch ist sowohl ein einfacher Austausch eines Endoskops gegen ein weiteres Endoskop im sterilen Umfeld als auch das Wiederverbinden eines bereits zuvor im sterilen Umfeld mit dem Kabel verbundenen Endoskops möglich. Dies ist insbesondere dann sinnvoll, wenn mehrere Endoskope mit unterschiedlichen optischen und/oder geometrischen Eigenschaften bei einer medizinischen Behandlung, insbesondere einer Operation, eingesetzt werden.

Besonders vorteilhaft ist es, wenn die zweite Sterilschleuse mit dem Kabel oder mit dem am Kabel vorhandenen Steckverbinder über eine lösbare Rastverbindung verbindbar ist. Hierdurch ist eine einfache und gemeinsame Handhabung beim Herstellen und Trennen einer Verbindung der Sterilschleuse und des Kabels bzw. dem am Kabel vorhandenen Steckverbinder mit dem Endoskop bzw. mit einem am Endoskop vorhandenen Steckverbinder möglich. Durch die lösbare Rastverbindung ist die Sterilschleuse so einfach mit dem Kabel oder mit dem am Kabel vorhandenen Steckverbinder verbindbar als auch nach dem Gebrauch wieder vom Kabel bzw. vom Steckverbinder trennbar.

Ferner ist es vorteilhaft, wenn die Endoskophülle eine Öffnung hat, in die das Endoskop einführbar ist, und wenn die Öffnung der Endoskophülle mit Hilfe eines

Verschlusselements steril verschließbar ist, wobei das Verschlusselement vorzugsweise die erste Sterilschleuse umfasst. Das Verschlusselement kann insbesondere als Platte oder Kappe ausgebildet sein, die die Öffnung der Endoskophülle verschließen. Das Verschlusselement kann über ein Filmscharnier und/oder eine Rastverbindung mit der Endoskophülle verbunden sein. Zwischen der Öffnung der Endoskophülle und dem Verschlusselement kann ferner ein Dichtbereich und/oder mindestens ein Dichtelement vorgesehen sein, der bzw. das die Verbindungsstelle zwischen dem Verschlusselement und der Öffnung der Endoskophülle fluiddicht abdichtet. Alternativ zur Rastverbindung oder Clickverbindung kann das Verschlusselement auch über eine andere geeignete formschlüssige oder kraftschlüssige Verbindung mit der übrigen Endoskophülle verbunden sein. Vorzugsweise umfasst die Endoskophülle das Verschlusselement mit der ersten Sterilschleuse, wobei die das Verschlusselement und die übrige Endoskophülle einteilig, vorzugsweise einstückig, sind. Einteilig bedeutet in diesem Zusammenhang, dass das Verschlusselement und die übrige Endoskophülle zu einem Bauteil verbunden sind, so dass sie gemeinsam gehandhabt werden können. Das Verschlusselement und die übrige Endoskophülle können jedoch getrennt gefertigt sein und dann miteinander verbunden worden sein. Die Verbindung kann insbesondere eine kraft-, stoff- oder formschlüssige Verbindung sein. Vorzugsweise ist bei der einteiligen Ausführung das Verschlusselement mit der übrigen Endoskophülle derart verbindbar, dass diese Verbindung zerstörungsfrei lösbar ist. Bei einer einstückigen Ausführung, die eine Spezialform der einteiligen Ausführung ist, sind das Verschlusselement und die Endoskophülle nicht zerstörungsfrei trennbar. Vorzugsweise sind sie im Verbundenen Zustand hergestellt, insbesondere durch einen Gießvorgang, bei dem ein Filmscharnier zwischen dem Verschlusselement und der übrigen Endoskophülle ausgebildet wird. Die erste Sterilschleuse kann jedoch weitere separat gefertigte Bauelemente umfassen. Bei der einstückigen Ausführung sind das Verschlusselement und die Endoskophülle somit vorzugsweise nicht zerstörungsfrei voneinander trennbar.

Vorzugsweise ist das Kabel ein elektrisches Kabel mit mindestens einem elektrischen Leiter, ein optisches Kabel mit mindestens einem optischen Übertragungspfad, ein Lichtleiterkabel mit mindestens einem optischen Übertragungspfad oder ein Hybridkabel mit mindestens einem elektrischen Leiter und mindestens einem optischen Übertragungspfad. Der optische Übertragungspfad kann insbesondere mindestens eine optische Faser umfassen. Vorzugsweise koppelt der Steckverbinder den mindestens einen optischen Übertragungspfad des Kabels mit mindestens einem optischen Element des Endoskops. Alternativ oder zusätzlich kann der Steckverbinder die mindestens eine elektrische Leitung des Kabels mit mindestens einem elektrischen Anschluss des Endoskops verbinden. Über den Steckverbinder ist eine einfache Verbindung zwischen dem Endoskop und dem Kabel möglich, ohne dass für das Verbinden oder Trennen spezielle Kenntnisse oder Fähigkeiten erforderlich sind. Insbesondere braucht sich ein Benutzer beim Verbinden und Trennen keine Gedanken über die korrekte sterile Abdeckung von nicht sterilen Elementen machen, da die Klappen vorzugsweise in ihrer geschlossenen Position gehalten bzw. in diese zurückbewegt werden. Hierzu kann insbesondere ein elastisch verformtes Element, wie beispielsweise eine Feder oder ein Polymerblock, eingesetzt werden.

Ferner ist es vorteilhaft, wenn das Endoskop einen ersten Steckverbinder hat und wenn an mindestens einem Ende des Kabels ein zum ersten Steckverbinder komplementärer zweiter Steckverbinder vorgesehen ist, der mit dem ersten Steckverbinder des Endoskops verbindbar ist. Vorzugsweise ist mit Hilfe des ersten Steckverbinders und des zweiten Steckverbinders mindestens eine optische Faser des Kabels mit mindestens einem optischen Element des Endoskops koppelbar. Alternativ oder zusätzlich ist mit Hilfe des ersten Steckverbinders und des zweiten Steckverbinders mindestens eine elektrische Leitung des Kabels mit mindestens einem elektrischen Anschluss des Endoskops verbindbar. Besonders vorteilhaft ist es, wenn beim Verbinden des zweiten Steckverbinders des Kabels mit dem ersten Steckverbinder des Endoskops zwischen dem ersten Steckverbinder und dem zweiten Steckverbinder eine lösbare Rastverbindung hergestellt wird. Dadurch ist zum Trennen des zweiten Steckverbinders des Kabels vom ersten Steckverbinder des Endoskops lediglich die Rastverbindung zu lösen. Dadurch ist eine einfache Handhabung des Kabels und des Endoskops möglich. Die Rastverbindung kann insbesondere derart ausgebildet sein, dass zum Lösen der Rastverbindung kein Werkzeug notwendig ist, sondern dass die Rastverbindung durch eine Handbetätigung einfach lösbar ist.

Weiterhin ist es vorteilhaft, wenn die erste Sterilschleuse bei der Aufnahme des Endoskops in der Endoskophülle eine definierte Lage zu einer Koppelschnittstelle des Endoskops hat, wobei die Koppelschnittstelle des Endoskops mindestens einen elektrischen Anschluss des Endoskops oder ein optisches Element umfasst. Hierdurch wird erreicht, dass durch die definierte Lage der Endoskophülle zur Koppelschnittstelle des Endoskops das Kabel bzw. der mit dem Kabel verbundene Steckverbinder sicher mit dem Endoskop verbunden werden kann.

Ferner ist es vorteilhaft, wenn die zweite Sterilschleuse einen ersten Verbindungsbereich hat, mit dem das Kabel und/oder der Steckverbinder verbindbar ist und wenn die zweite Sterilschleuse einen zweiten Verbindungsbereich hat, mit dem ein komplementärer Verbindungsbereich der ersten Sterilschleuse verbindbar ist. Hierdurch kann der Steckverbinder oder das Kabel in der zweiten Sterilschleuse unabhängig von einer Verbindung der ersten Sterilschleuse mit der zweiten Sterilschleuse verbunden sein. Dies vereinfacht die Handhabung des Kabels und erleichtert die Verbindung des Kabels mit dem Endoskop über die beiden Sterilschleusen. Vorzugsweise sind der erste Verbindungsbereich der zweiten Sterilschleuse und der zweite Verbindungsbereich der zweiten Sterilschleuse auf einander abgewandten Seiten der zweiten Sterilschleuse angeordnet. Hierdurch kann die Verbindung des Kabels bzw. Steckverbinders mit dem ersten Verbindungsbereich unabhängig von einer Verbindung zwischen der ersten Sterilschleuse und dem zweiten Verbindungsbereich der zweiten Sterilschleuse erfolgen. Auch kann der zweite Verbindungsbereich der zweiten Sterilschleuse unabhängig davon mit der ersten Sterilschleuse verbunden werden, ob mit dem zweiten Verbindungsbereich der zweiten Sterilschleuse das Kabel oder der Steckverbinder verbunden ist.

Dabei ist es vorteilhaft, wenn der erste Verbindungsbereich der zweiten Sterilschleuse über eine erste lösbare Rastverbindung mit dem Kabel und/oder dem am Ende des Kabels vorgesehenen Steckverbinder verbindbar ist und wenn der zweite Verbindungsbereich der zweiten Sterilschleuse über eine zweite lösbare Rastverbindung mit dem Verbindungsbereich der ersten Sterilschleuse verbindbar ist. Hierdurch sind die Verbindungen zwischen den Sterilschleusen und zwischen der zweiten Sterilschleuse und dem Steckverbinder bzw. der zweiten Sterilschleuse und dem Kabel vor unbeabsichtigten Trennen mit Hilfe der Rastverbindung gesichert. Andererseits sind sie durch einfaches Lösen der Rastverbindung im Bedarfsfall voneinander trennbar.

Besonders vorteilhaft ist es, wenn die erste Sterilklappe beim Verbinden der ersten Sterilschleuse mit der zweiten Sterilschleuse automatisch öffnet und wenn die zweite Sterilklappe beim Verbinden der ersten Sterilschleuse mit der zweiten Sterilschleuse automatisch öffnet, wenn die erste Sterilklappe beim Trennen der ersten Sterilschleuse von der zweiten Sterilschleuse automatisch schließt und wenn die zweite Sterilklappe beim Trennen der ersten Sterilschleuse von der zweiten Sterilschleuse automatisch schließt. Hierdurch wird sichergestellt, dass keine Bedienhandlungen einer Bedienperson zum sterilen Abdecken von nicht sterilen Elementen beim Trennen der Sterilschleusen voneinander erforderlich sind, so dass hierdurch ein einem hohen Maß sichergestellt werden kann, dass der sterile Bereich nicht infolge von Bedienfehlern kontaminiert wird.

Besonders vorteilhaft ist es hierbei, wenn beim Verbinden der ersten Sterilschleuse mit der zweiten Sterilschleuse eine automatische Entriegelung der ersten Sterilklappe erfolgt, wenn beim Verbinden der ersten Sterilschleuse mit der zweiten Sterilschleuse eine automatische Entriegelung der zweiten Sterilschleuse erfolgt, wenn beim Trennen der ersten Sterilschleuse von der zweiten Sterilschleuse eine automatische Verriegelung der ersten Sterilklappe erfolgt und wenn beim Trennen der ersten Sterilschleuse von der zweiten Sterilschleuse eine automatische Verriegelung der zweiten Sterilklappe erfolgt. Hierdurch wird sichergestellt, dass sich die Klappen im getrennten Zustand bzw. im nicht verbundenen Zustand der ersten Sterilschleuse und der zweiten Sterilschleuse in ihrer geschlossenen Position verriegelt sind, so dass diese nicht versehentlich geöffnet werden können: Hierdurch wird die mit dem versehentlichen Öffnen verbundene Gefahr der Kontamination des sterilen Bereichs sicher ausgeschlossen.

Ferner ist es vorteilhaft, wenn die sterile Außenseite der ersten Sterilklappe beim Verbinden der ersten Sterilschleuse mit der zweiten Sterilschleuse an der sterilen Außenseite der zweiten Sterilklappe gegenüberliegend angeordnet ist, wenn sowohl die erste Sterilklappe als auch die zweite Sterilklappe geöffnet sind. Die Außenseite der ersten Sterilklappe und die Außenseite des zweiten Verbindungsbereichs sind im geöffneten Zustand einander zugewandt. Hierdurch wird sicher verhindert, dass die bei der Handhabung der Sterilschleusen mit nicht sterilen Elementen in Kontakt gebrachten Innenseiten der Verbindungsbereiche nicht die sterilen Außenseiten der anderen Verbindungsbereiche kontaminieren, so dass eine nachfolgend mögliche Kontamination des sterilen Bereichs durch die anderenfalls kontaminierte Außenseite der Verbindungsbereiche ausgeschlossen ist.

Ferner ist es vorteilhaft, einen Abschnitt der Endoskophülle einen Schaft eines als Stabendoskop ausgebildeten Endoskops aufzunehmen und vorzugsweise zu umschließen. Die Endoskophülle kann ein optisches Element umfassen, das dem Schaftende des Endoskops gegenüberliegend angeordnet ist, wenn das Endoskop in der Endoskophülle aufgenommen ist. Im einfachsten Fall ist das optische Element als lichtdurchlässiges, das Licht nicht formendes Element, ausgebildet. Hierbei kann es sich um eine einfache Sichtscheibe handeln. Alternativ kann das optische Element als Linse oder Prisma ausgebildet sein.

Bei dem Vorsehen von unterschiedlichen optischen Elementen in verschiedenen Endoskophüllen kann über die Wahl der Endoskophülle die Abbildung des mit Hilfe des Endoskops detektierten Lichts verändert werden, so dass die optischen Bildaufnahmeeigenschaften des Endoskops durch die Wahl der Hülle verändert werden können.

Ein zweiter Aspekt der Erfindung betrifft eine Anordnung zur robotergestützten Chirurgie. Diese Anordnung eignet sich insbesondere zu einer telerobotergestützten Prozedur innerhalb eines sterilen Bereichs. Die Anordnung umfasst mindestens eine Anordnung nach Anspruch 1 oder eine zuvor angegebene Weiterbildung der Anordnung nach Anspruch 1. Ferner umfasst die Anordnung mindestens einen Manipulator, der einen Manipulatorarm hat. Ferner umfasst die Anordnung mindestens eine Eingabeeinrichtung zur Eingabe mindestens eines Eingabekommandos. Das in der Endoskophülle aufgenommene Endoskop ist mit dem Manipulatorarm verbindbar. Die Anordnung umfasst ferner eine Steuereinheit, die dem Manipulatorarm und das mit dem Manipulatorarm verbundene Endoskop abhängig von dem Eingabekommando mit Hilfe mindestens einer Antriebseinheit positioniert. Dadurch ändert der Manipulatorarm vorzugsweise abhängig von dem Eingabekommando die Position des Endoskops im Raum. Hierdurch kann die Position des Endoskops einfach mit Hilfe eines Telemanipulators geändert werden, so dass auch während einer Operation mit Hilfe eines Telemanipulatorsystems die Blickrichtung und die Position des Endoskops einfach über Bedieneingaben über die Eingabeeinrichtung geändert werden können. Vorzugsweise kann über dieselbe Eingabeeinrichtung ein chirurgisches Instrument, das mit einem weiteren Manipulatorarm des Manipulators verbunden ist, bedient werden und dessen Lage im Raum mit Hilfe des Manipulatorarms geändert werden.

Ein dritter Aspekt der Erfindung betrifft eine Anordnung zum Herstellen und Lösen einer Steckverbindung zwischen einem ersten nicht sterilen Steckverbinder und einem zum ersten Steckverbinder komplementären zweiten nicht sterilen Steckverbinder in einer sterilen Umgebung. Die Anordnung umfasst eine erste sterile Hülle zur Aufnahme des ersten Steckverbinders und eine zweite sterile Hülle zur Aufnahme zumindest des zweiten Steckverbinders Die erste Hülle umfasst eine erste Sterilschleuse, die mindestens eine erste Sterilklappe hat, die im geschlossenen Zustand den ersten Steckverbinder steril abschirmt. Die zweite Hülle umfasst eine zweite Sterilschleuse, die mindestens eine zweite Sterilklappe hat, die im geschlossenen Zustand den zweiten Steckverbinder steril abschirmt. Beim Verbinden des ersten Steckverbinders mit dem zweiten Steckverbinder erfolgt eine Bewegung der ersten Sterilklappe von ihrem geschlossenen Zustand in einen geöffneten Zustand. Ferner erfolgt beim Verbinden des ersten Steckverbinders mit dem zweiten Steckverbinder eine Bewegung der zweiten Sterilklappe von ihrem geschlossenen Zustand in einen geöffneten Zustand, so dass eine direkte Kopplung des ersten Steckverbinders mit dem zweiten Steckverbinder möglich ist. Beim Trennen des ersten Steckverbinders von dem zweiten Steckverbinder erfolgt eine Bewegung der ersten Sterilklappe von dem geöffneten Zustand in den geschlossenen Zustand sowie eine Bewegung der zweiten Sterilklappe von dem geöffneten Zustand in den geschlossenen Zustand, so dass die erste Sterilklappe nach dem Trennen zumindest den ersten Steckverbinder steril abschirmt und die zweite Sterilklappe nach dem Trennen den zweiten Steckverbinder steril abschirmt.

Durch eine solche Anordnung ist es einfach möglich, nicht sterile Steckverbinder steril abzuschirmen, so dass sie ohne besondere Vorsicht in einer sterilen Umgebung verwendet werden können. Darüber hinaus wird sichergestellt, dass eine direkte Verbindung zwischen den Steckverbindern hergestellt werden kann, indem beim Verbinden der Steckverbinder die Sterilklappen der ersten Sterilschleuse und der zweiten Sterilschleuse jeweils geöffnet werden. Ferner ist sichergestellt, dass nach dem Trennen der Steckverbinder diese wieder steril abgeschirmt werden, indem die Sterilklappen der Sterilschleusen wieder geschlossen werden. Somit ist ein Trennen und Verbinden sowie ein Wiederverbinden der Steckverbinder in der sterilen Umgebung problemlos möglich, so dass dies auch während einer Operation erforderlichenfalls durchgeführt werden kann.

Ferner ist es bei allen Aspekten oder deren Weiterbildungen vorteilhaft, wenn der erste Steckverbinder mindestens ein erstes elektrisches Kontaktelement zum Herstellen einer elektrischen Verbindung oder mindestens ein erstes optisches Element zum Herstellen einer optischen Verbindung hat, wenn der zweite Steckverbinder mindestens ein zweites elektrisches Kontaktelement zum Herstellen einer zweiten elektrischen Verbindung mit dem ersten Kontaktelement oder mindestens ein zweites optisches Element zum Herstellen einer optischen Verbindung mit dem optischen Element hat, wenn der mindestens eine erste elektrische Kontakt und der mindestens eine zweite elektrische Kontakt und/oder wenn das mindestens eine erste optische Element und das mindestens eine zweite optische Element derart angeordnet und ausgebildet ist, dass bei einer Verbindung zwischen dem ersten Steckverbinder und dem zweiten Steckverbinder eine elektrische Verbindung zwischen dem ersten Kontaktelement und dem zweiten Kontaktelement oder eine optische Verbindung zwischen dem ersten optischen Element und dem zweiten optischen Element erfolgt. Der erste Steckverbinder und/oder der zweite Steckverbinder können dabei fest mit einem medizinischen Gerät verbunden sein, insbesondere mit dem Gehäuse eines medizinischen Gerätes. Bei dem medizinischen Gerät kann es sich insbesondere um ein Endoskop oder ein chirurgisches Instrument handeln, die insbesondere zusammen mit einem Manipulator, vorzugsweise bei der robotergestützten Chirurgie, verwendet werden können.

Ferner ist es vorteilhaft, wenn der erste Steckverbinder und der zweite Steckverbinder derart symmetrisch ausgebildet sind, dass die elektrische Verbindung zwischen dem ersten Kontaktelement und dem zweiten Kontaktelement und/oder die optische Verbindung zwischen dem ersten optischen Element und dem zweiten optischen Element sowohl beim Zusammenführen der Steckverbinder in einer ersten Position und beim Zusammenführen der Steckverbinder in einer zweiten Position möglich ist, wobei in der zweiten Position einer der Steckverbinder um eine senkrecht zur Kontaktebene der Steckverbinder verlaufende Achse um 180 Grad gedreht worden ist. Hierbei ist eine einfache intuitive Verbindung möglich, da insbesondere eine Verbindung der Steckverbinder in zwei um 180 Grad versetzte Positionen möglich ist, so dass nicht auf Details der Steckverbinder vor dem Zusammenführen geachtet werden muss, sondern vielmehr ein intuitives Zusammenführen der Steckverbinder möglich ist.

Weitere Merkmale und Vorteile der Erfindung ergeben sich aus der folgenden Beschreibung, die die Erfindung anhand von Ausführungsbeispielen im Zusammenhang mit den beigefügten Figuren näher erläutert.

Es zeigen:
- Figur 1: eine schematische Schnittdarstellung einer Anordnung mit einem in einer Endoskophülle aufgenommenen Endoskop und mit einem in einer Kabelhülle aufgenommenen Endoskopkabel in einem nicht verbundenen Zustand gemäß einer ersten Ausführungsform;
- Figur 2: die Anordnung nach Figur 1, wobei das Endoskop und das Endoskopkabel in einem verbundenen Zustand dargestellt sind;
- Figur 3: eine Anordnung mit einem in einer Endoskophülle aufgenommenen Endoskop und einem mit einem Steriladapter verbundenen Steckverbinder eines Endoskopkabels, bei der eine lösbare Verbindung zwischen Endoskopkabel und Endoskop herstellbar ist, gemäß einer zweiten Ausführungsform;
- Figur 4: die Endoskophülle und ein mit Hilfe der Endoskophülle steril abschirmbares Endoskop in einem noch nicht verbundenen Zustand;
- Figur 5: ein Abschnitt der Endoskophülle mit eingeführtem Endoskop mit geöffneter Einführöffnung zum Einführen und Entnehmen des Endoskops, wobei die Endoskophülle ein Verschlusselement zum Verschließen der Einführöffnung umfasst, in das eine erste Sterilschleuse integriert ist;
- Figur 6: die Kabelhülle zum sterilen Abschirmen zumindest eines Teils des Endoskopkabels, wobei die Kabelhülle ein Steriladapter umfasst, mit dem ein am Kabelende vorgesehener Steckverbinder verbindbar ist und das eine zweite Sterilschleuse umfasst;
- Figur 7a: eine Seitenansicht eines Abschnitts des in der Endoskophülle aufgenommenen Endoskops und des mit dem Steriladapter der Kabelhülle verbundenen Steckverbinders des Endoskopkabels in einer ersten Position vor dem Zusammenführen;
- Figur 7b: eine Schnittdarstellung der Anordnung nach Figur 7a;
- Figur 8a: die Anordnung nach Figur 7a in einer zweiten Position beim Zusammenführen des Steckverbinders des Endoskopkabels mit einem Steckverbinder des Endoskops;
- Figur 8b: eine Schnittdarstellung der Anordnung nach Figur 8a;
- Figur 9a: die Anordnung nach den Figuren 7a und 8a in einer dritten Position, in der der Steckverbinder des Endoskopkabels und der Steckverbinder des Endoskops weiter angenähert sind;
- Figur 9b: eine Schnittdarstellung der Anordnung nach Figur 9a;
- Figur 10a: die Anordnung nach den Figuren 7a, 8a, 9a in einer weiteren Position, in der der Steckverbinder des Endoskopkabels und der Steckverbinder des Endoskops weiter angenähert sind und die Koppelelemente zum Herstellen einer optischen Verbindung zwischen dem Endoskopkabel und dem Endoskop mit Hilfe von Führungsmitteln positioniert werden,
- Figur 10b: eine Schnittdarstellung der Anordnung nach Figur 10a;
- Figur 11a: die Anordnung nach den Figuren 7a bis 10a in einer Position, in der der Steckverbinder des Endoskopkabels und der Steckverbinder des Endoskops in einem verbundenen Zustand angeordnet sind;
- Figur 11b: eine Schnittdarstellung der Anordnung nach Figur 11a;
- Figur 12a: eine Explosionsdarstellung der Elemente der Endoskophülle und der Elemente der Kabelhülle mit geöffneten Sterilklappen;
- Figur 12b: die Explosionsdarstellung nach Figur 12a mit geschlossenen Sterilklappen;
- Figur 13: eine perspektivische Ansicht eines Abschnitts des Endoskops mit dem Steckverbinder des Endoskops;
- Figur 14: eine perspektivische Ansicht eines Abschnitts des Endoskopkabels zusammen mit dem am Kabelende angeordneten Steckverbinder;
- Figur 15a: eine erste perspektivische Ansicht der Kabelhülle mit dem Steriladapter der Kabelhülle;
- Figur 15b: eine zweite perspektivische Ansicht der Kabelhülle mit dem Steriladapter der Kabelhülle;
- Figur 16: die Endoskophülle und das Verschlusselement der Endoskophülle in einem geöffneten Zustand;
- Figur 17: eine schematische Seitenansicht eines Systems zur robotergestützten Chirurgie mit einem Manipulator, der vier Manipulatorarme hat, mit denen jeweils eine Instrumenteneinheit verbindbar ist; und
- Figur 18: eine schematische Vorderansicht des Systems nach Figur 17.

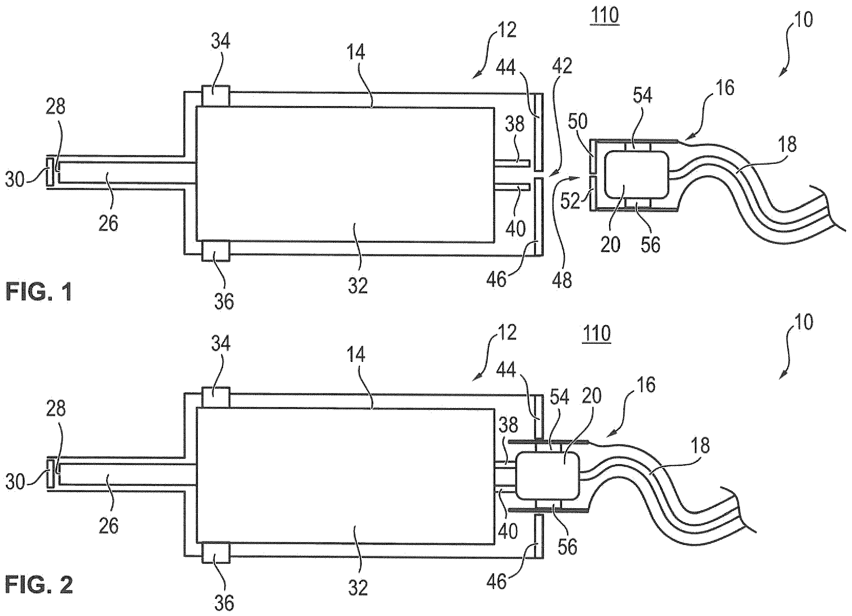

Figur 1 zeigt eine schematische Schnittdarstellung einer Anordnung 10 mit einem in einer Endoskophülle 12 aufgenommene Endoskope 14. Die Anordnung 10 umfasst ferner ein von einer Kabelhülle 16 umschlossenes Endoskopkabel 18. Das Endoskopkabel 18 dient zum Verbinden des Endoskops 14 mit einer nicht dargestellten Steuereinheit zur Steuerung des Endoskops 14 und/oder zur Verarbeitung der vom Endoskop 14 übertragenen Bilddaten. Am endoskopseitigen Ende des Endoskopkabels 18 ist ein Steckverbinder 20 vorgesehen, mit dessen Hilfe das Endoskopkabel 18 einfach mit dem Endoskop 14 verbindbar und wieder trennbar ist. Die Endoskophülle 12 dient zum sterilen Abschirmen des nicht sterilen Endoskops 14 in einem sterilen Bereich 110, d.h. einer sterilen Umgebung. Die Kabelhülle 16 dient zum Abschirmen des nicht sterilen Endoskopkabels 18 und des mit dem Endoskopkabel 18 verbundenen Steckverbinders 20.

Bei dem Endoskop 14 handelt es sich um ein Stabendoskop, das einen zumindest teilweise in einen Patientenkörper einführbaren stabförmigen Bereich 26 hat, in dem nicht dargestellte optische Elemente zum Leiten von Beleuchtungslicht und zur Detektion und/oder Weiterleitung von in die Spitze 28 des stabförmigen Bereichs 26 eintretendes Umgebungslicht umfasst. Der Spitze 28 gegenüberliegend ist in der Endoskophülle 12 ein optisches Element vorgesehen, das zumindest einen Teil des abzustrahlenden Beleuchtungslichts durchlässt und zumindest einen Teil des Umgebungslichts zur Spitze 28 des stabförmigen Bereichs 26 durchlässt. Im einfachsten Fall ist das optische Element eine Sichtscheibe bzw. ein Fenster. Bei anderen Ausführungsbeispielen kann das optische Element eine Linse sein oder ein Prisma oder eine Kombination aus mehreren einzelnen optischen Elementen.

Das Endoskop 14 umfasst ferner einen Endoskopkörper 32 in dem insbesondere eine Lichtquelle zum Erzeugen von Beleuchtungslicht und eine Steuereinheit zum Erfassen und Verarbeiten von mit Hilfe eines Bildsensors aufgenommenen Bildern bzw. zur Verarbeitung der bei der Bildaufnahme erzeugten Bilddaten, umfasst. Im vorliegenden Ausführungsbeispiel sind mechanische Verbindungselemente 34, 36 vorgesehen, durch die das Endoskop 14 in der Endoskophülle 12 fixierbar ist. Im vorliegenden Fall sind die mechanischen Verbindungselemente 34, 36 durch einen in die Endoskophülle 12 integrierten Passring gebildet. Bei anderen Ausführungsbeispielen können auch andere mechanische Verbindungselemente, wie Schrauben, Klemm- oder Rastelemente vorgesehen sein. Alternativ oder zusätzlich kann die Endoskophülle 12 derart ausgebildet sein, dass sie das Endoskop 14 insgesamt so eng umschließt, so dass das Endoskop 14 in seiner Lage relativ zur Endoskophülle 12 fixiert ist.

Das Endoskop 14 hat mindestens einen elektrischen Kontakt 38 zum Herstellen einer elektrischen Verbindung zwischen dem Endoskop 14 und mindestens einer elektrischen Leitung des Endoskopkabels 18. Mit Hilfe dieses elektrischen Kontakts 38 kann insbesondere elektrische Energie zum Endoskop 14 übertragen werden, um insbesondere eine im Endoskop 14 angeordnete Steuereinheit mit elektrischer Energie zu versorgen und/oder eine im Endoskop 14 vorhandene Lichtquelle mit elektrischer Energie zu versorgen. Alternativ oder zusätzlich können über den elektrischen Kontakt 38 oder über weitere elektrische Kontakte Daten, insbesondere Bilddaten, vom Endoskop 14 über das Endoskopkabel 18 zu einer externen Steuer- und/oder Verarbeitungseinheit übertragen werden. Alternativ oder zusätzlich können auch Daten von einer solchen externen Steuereinheit zum Endoskop 14 übertragen werden, um insbesondere die Funktion des Endoskops 14, beispielsweise die Helligkeit oder das Spektrum des Beleuchtungslichts zu steuern oder um beispielsweise eine Zoomfunktion des Endoskops 14 zu aktivieren. Darüber hinaus umfasst das Endoskop 14 ein optisches Verbindungselement 40, das mit einem komplementären optischen Verbindungselement des Steckverbinders 20 zur optischen Datenübertragung, zur Übertragung von Beleuchtungslicht oder zur Übertragung von optischen Bildinformationen vom Endoskop zu einer externen Steuer- und/oder Auswerteieinheit über das Endoskopkabel 18.

Die Endoskophülle 12 umfasst eine erste Sterilschleuse 42 mit einem ersten Sterilklappenpaar mit den Sterilklappen 44, 46. Mit Hilfe der Sterilklappen 44, 46 wird der Kontaktbereich des Endoskops 14 mit dem elektrischen Kontakt 38 und dem optischen Verbindungselement 40 steril abgeschirmt. Die Kabelhülle 16 des Endoskopkabels 18 umfasst eine zweite Sterilschleuse 48 mit den Sterilklappen 50, 52, wobei der Steckverbinder 20 des Endoskopkabels 18 mit Hilfe von mechanischen Verbindungselementen 54, 56 mit dem Gehäuse der Sterilschleuse 48 verbunden sind, so dass der Steckverbinder 20 in seiner Lage relativ zu den Sterilklappen 50, 52 in der Sterilschleuse 48 fixiert ist. Mit Hilfe der Sterilklappen 50, 52 der Sterilschleuse 48 wird der nicht sterile Steckverbinder 20 gegenüber dem sterilen Bereich 110 abgeschirmt. In Figur 1 sind der Steckverbinder 20 und das Endoskop 14 in einem getrennten Zustand gezeigt, in dem sowohl die Sterilklappen 44, 46 der ersten Sterilschleuse 42 als auch die Sterilklappen 50, 52 der zweiten Sterilschleuse 48 geschlossen sind.

Figur 2 zeigt die Anordnung nach Figur 1, wobei das Endoskop 14 mit dem Endoskopkabel 18 in einem verbundenen Zustand dargestellt ist. Ausgehend von der in Figur 1 dargestellten Position sind das in der Endoskophülle 12 befindliche Endoskop 14 und das in der Kabelhülle 16 befindliche Endoskopkabel 18 aufeinander zubewegt worden, bis sie den in Figur 2 dargestellten Zustand erreicht haben. Während des Aufeinanderzubewegens kommen die erste Sterilschleuse 42 der Endoskophülle 12 und die zweite Sterilschleuse 48 der Kabelhülle 16 in Kontakt miteinander. Dabei werden sowohl die Schleusenklappen 44,46 der ersten Sterilschleuse 42 als auch die Schleusenklappen 50, 52 der zweiten Sterilschleuse 48 entriegelt und geöffnet. Dadurch ist eine direkte Verbindung zwischen dem elektrischen Kontakt 38 und dem optischen Verbindungselement 40 und entsprechend komplementären Verbindungselementen bzw. Kontaktelementen des Steckverbinders 20 möglich ist. Diese direkte Verbindung wird automatisch hergestellt, wenn der Steckverbinder 20 des Endoskopkabels 18 und das Endoskop 16 nach dem Öffnen der Schleusenklappen 44,46, 52, 54 weiter aufeinander zubewegt werden. Vorzugsweise sind das elektrische Kontaktelement 38 und das optische Verbindungselement 40 Bestandteil eines am Endoskop 14 vorgesehenen Steckverbinders, der komplementär zum Steckverbinder 20 des Endoskopkabels 18 ist. Hierdurch ist eine einfache und sichere elektrische und/oder optische Verbindung zwischen dem Endoskop 14 und dem Endoskopkabel 18 möglich.

Zum Trennen des in der Kabelhülle 16 angeordneten Endoskopkabels 18 von dem in der Endoskophülle 12 angeordnete Endoskop 16 werden diese voneinander wegbewegt, wobei sowohl die Schleusenklappen 44,46 der ersten Sterilschleuse 42 als auch die Schleusenklappen 50, 52 der zweiten Sterilschleuse 48 wieder automatisch geschlossen und verriegelt werden, so dass sich die Anordnung 10 nach dem Trennen wieder in dem in Figur 1 gezeigten Zustand befindet. Der sterile Bereich 110 war somit sowohl vor dem Verbinden, während des Verbindens, während des Trennens und nach dem Trennen vor einer Kontamination durch die nicht sterilen Elemente des Endoskops 14 und des Kabels 18 durch die sterile Endoskophülle 12 mit der ersten Sterilschleuse 42 und durch die sterile Kabelhülle 16 mit der zweiten Sterilschleuse 48 geschützt.

Das in der Kabelhülle 18 angeordnete Endoskopkabel 16 kann auf die zuvor beschriebene Weise beliebig oft im sterilen Bereich 110 mit dem in der Endoskophülle 12 angeordneten Endoskop 14 oder alternativ mit einem weiteren in einer sterilen Endoskophülle angeordneten Endoskop verbunden und von diesem wieder getrennt werden, ohne dass die Gefahr besteht, dass der sterile Bereich 110 kontaminiert wird.

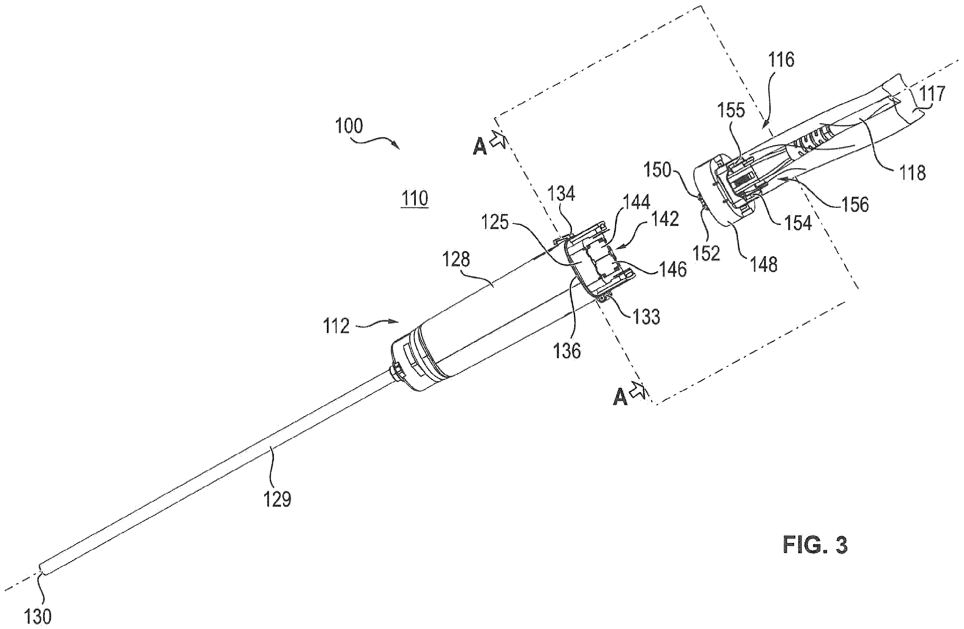

Figur 3 zeigt eine Anordnung 100 mit einem in einer Endoskophülle 112 aufgenommenen Endoskop. Das in der Endoskophülle 112 aufgenommene Endoskop ist in Figur 3 nicht sichtbar. Ferner umfasst die Anordnung 100 einen sterilen Schlauch 116 zum Umhüllen eines Endoskopkabels 118. Der sterile Schlauch 116 umfasst einen Steriladapter 148. Das Endoskopkabel 118 hat an seinem zur Verbindung mit dem in der Endoskophülle 112 aufgenommenen Endoskop vorgesehenen Ende einen Steckverbinder 156, der über einen Rastverbinder 154 mit dem Steriladapter 148 lösbar verbunden ist. Bei der Anordnung 100 nach Figur 3 ist eine lösbare Verbindung zwischen dem in der Kabelhülle 116 aufgenommenen Endoskopkabel 118 und dem in der Endoskophülle 112 aufgenommenen Endoskop herstellbar. Das in der Endoskophülle 112 befindliche nicht sterile Endoskop und das in der Kabelhülle 116 angeordnete Endoskopkabel 118 befinden sich in einem sterilen Bereich 110, wie einem Operationssaal. Durch die Endoskophülle 112 ist das Endoskop und durch die Kabelhülle ist das Endoskopkabel 118 gegenüber dem sterilen Bereich 110 abgeschirmt.

Die Endoskophülle 112 umfasst einen vorderen Teil 129, der zur Aufnahme eines zumindest teilweise in einen Patientenkörper einführbaren Endoskopstabs oder Endoskopschlauchs hat. Der vordere Teil 129 der Endoskophülle 112 ist an seinem distalen Ende mit Hilfe eines optischen Elements 130 verschlossen. Das optische Element 130 kann eine Linse, ein Prisma oder eine den Strahlenverlauf nicht beeinflussende Scheibe umfassen.

Die Endoskophülle 112 umfasst ferner ein Mittelteil 128 zur Aufnahme eines Endoskopgehäuses und ein über ein Scharnier 133 mit dem Mittelteil 128 verbundenes Verschlusselement 125 mit einer Sterilschleuse 142. Durch das Verschlusselement 125 ist eine Zuführ- und Entnahmeöffnung 136 der Endoskophülle 112 zum Einführen und Herausnehmen eines Endoskops in bzw. aus der Endoskophülle 112 steril verschließbar ist. Das Verschlusselement 125 ist im geschlossenen Zustand mit Hilfe eines Rastverbinders 134, der auf der dem Scharnier 133 gegenüberliegenden Seite der Zuführ- und Entnahmeöffnung 136 der Endoskophülle 112 vorhanden ist, verriegelbar. Die in dem Verschlusselement 125 vorgesehene Sterilschleuse 142 hat Sterilklappen 144, 146. Das im Mittelteil 128 der Endoskophülle 112 angeordnete Endoskopgehäuse wird auch als Endoskopkörper bezeichnet, in dem insbesondere eine Lichtquelle zum Erzeugen von Beleuchtungslicht und eine Steuereinheit zum Erfassen und Verarbeiten von mit Hilfe eines Bildsensors aufgenommenen Bildern bzw. zur Verarbeitung der bei der Bildaufnahme erzeugten Bilddaten, vorgesehen sein kann. Ferner können mechanische Verbindungselemente vorgesehen sein, durch die das Endoskop in der Endoskophülle 112 fixierbar ist. Die mechanischen Verbindungselemente können beispielsweise durch einen in die Endoskophülle 112 integrierten Passring gebildet werden. Bei anderen Ausführungsformen können auch andere mechanische Verbindungselemente, wie Schrauben, Klemm- oder Rastelemente vorgesehen sein. Alternativ oder zusätzlich kann die Endoskophülle 112 derart ausgebildet sein, dass sie das Endoskop insgesamt so eng umschließt, so dass das Endoskop in seiner Lage relativ zur Endoskophülle 112 fixiert ist.

Der Steriladapter 148 des Kabelschlauchs 116 umfasst die Sterilklappen 150, 152 und wird auch als Sterilschleuse bezeichnet. In dem in Figur 3 gezeigten verschlossenen Zustand der Sterilklappen 144, 146, 150, 152 sind diese verriegelt. Beim Zusammenführen des Steriladapters 148 und der Sterilschleuse 142 werden die Sterilklappen 144, 146, 150, 152 automatisch entriegelt und geöffnet.

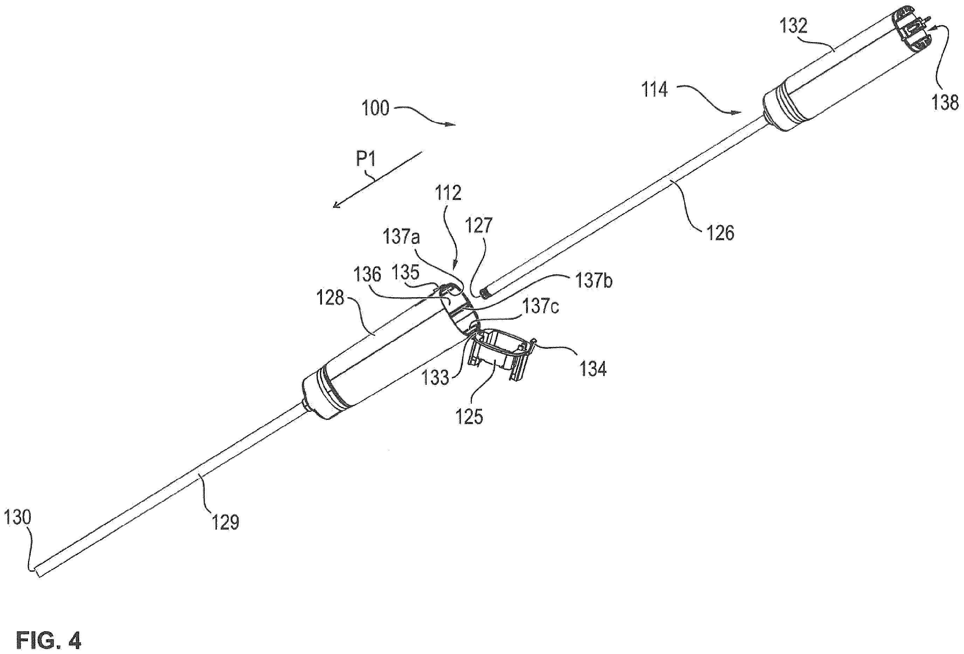

Figur 4 zeigt die Endoskophülle 112 und ein mit Hilfe der Endoskophülle 112 steril abschirmbares Endoskop 114 in einem noch nicht verbundenen Zustand. Zur Aufnahme des Endoskops 114 in der Endoskophülle 112 wird das Endoskop 114 in Richtung des Pfeils P1 durch die geöffnete Zuführ- und Entnahmeöffnung 136 in die Endoskophülle 112 eingeführt. Hierzu wird der stabförmige Bereich 126 des Endoskops 114 zuerst in die Zuführ- und Entnahmeöffnung 136 eingeführt und nachfolgend bis in den vorderen Teil 129 der Endoskophülle 112 geschoben, so dass die Spitze 127 des stabförmigen Bereichs 126 des Endoskops 114 dem am Ende des vorderen Teils 129 vorhandenen optischen Element 130 der Endoskophülle 112 gegenüberliegend angeordnet ist. Beim Einführen des Endoskopkörpers 132 durch die Zuführ- und Entnahmeöffnung 136 in das Mittelteil 128 der Endoskophülle 112 wird der Endoskopkörper 132 durch innen im Mittelteil 128 der Endoskophülle 112 vorhandene Führungsstege 137a bis 137f geführt und in einer vorbestimmten Lage im Mittelteil 128 der Endoskophülle 112 gehalten. An dem dem stabförmigen Bereich 126 gegenüberliegenden Endes des Endoskopkörpers 132 ist ein Hybridsteckverbinder 138 angeordnet, der sowohl optische Elemente zur Übertragung von optischen Signalen und/oder Beleuchtungslicht und/oder zur Übertragung von Bildern umfasst. Ferner umfasst der Hybridsteckverbinder 138 elektrische Kontakte zur Übertragung von elektrischer Energie zur Versorgung des Endoskops 114 mit elektrischer Energie und/oder zur Übertragung von Signalen und/oder Daten vom und zum Endoskop 114. Diese Daten können insbesondere Bilddaten, die ausgehend von mit Hilfe eines im Endoskop 114 vorhandenen Bildsensors aufgenommenen Bildern erzeugt worden sind und/oder Steuerdaten/Steuersignale zum Steuern des Endoskops 114 umfassen. Der Hybridsteckverbinder 138 ist komplementär zu dem auch als Hybridsteckverbinder ausgebildeten Steckverbinder 156 des Endoskopkabels 118, wobei die elektrischen Kontakte und die optischen Elemente des Steckverbinders 156 komplementär zu den elektrischen Kontakten und den optischen Elementen des Steckverbinders 138 sind.

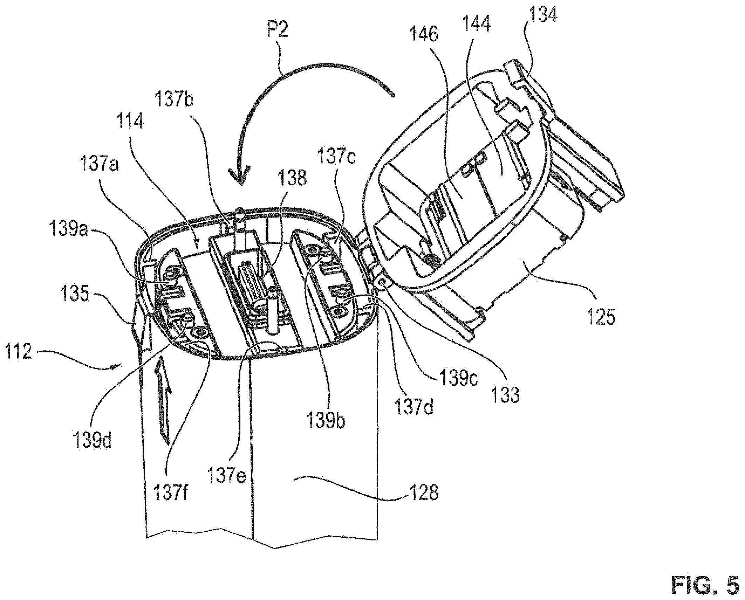

Figur 5 zeigt einen Abschnitt der Endoskophülle 112, wobei das Endoskop 114 in die Endoskophülle 112 eingeführt worden ist und sich in dieser befindet. Die Zuführ- und Entnahmeöffnung 136 ist bei der in Figur 5 dargestellten Anordnung geöffnet und kann durch Verschwenken des Verschlusselements 125 um die Drehachse des Scharniers 133 in Richtung des Pfeils P2 durch das Verschlusselement 125 verschlossen werden. An den Rastverbinder 134 greift in ein am Mittelteil 128 der Endoskophülle 112 ausgebildetes Eingriffselement 135 ein, wenn die Zuführ- und Entnahmeöffnung 136 durch das Verschlusselement 125 verschlossen ist. Hierdurch ist die Zuführ- und Entnahmeöffnung 136 sicher steril verschlossen. Der Rastverbinder 134 kann ferner insbesondere durch eine Bedienperson betätigt werden, um den Eingriff des Rastverbinders 134 mit dem Eingriffselement 135 zu lösen und dann das Verschlusselement 125 um die Drehachse des Scharniers 133 entgegen dem Pfeil P2 zu verschwenken. Dadurch wird die Zuführ- und Entnahmeöffnung 136 wieder geöffnet, so dass das Endoskop 114 wieder aus der Endoskophülle 112 entfernt werden kann, beispielsweise nach einer Operation. An dem entgegengesetzten Ende der Spitze 127 des Endoskops 114 sind neben dem Hybridsteckverbinder 138 mehrere Distanzelemente 139a bis 139d angeordnet, die das Verschlusselement 125 im geschlossenen Zustand kontaktieren, so dass das Endoskop 114 einen definierten Abstand und somit eine definierte Lage zu der Sterilschleuse 142 des Verschlusselements 125 hat.

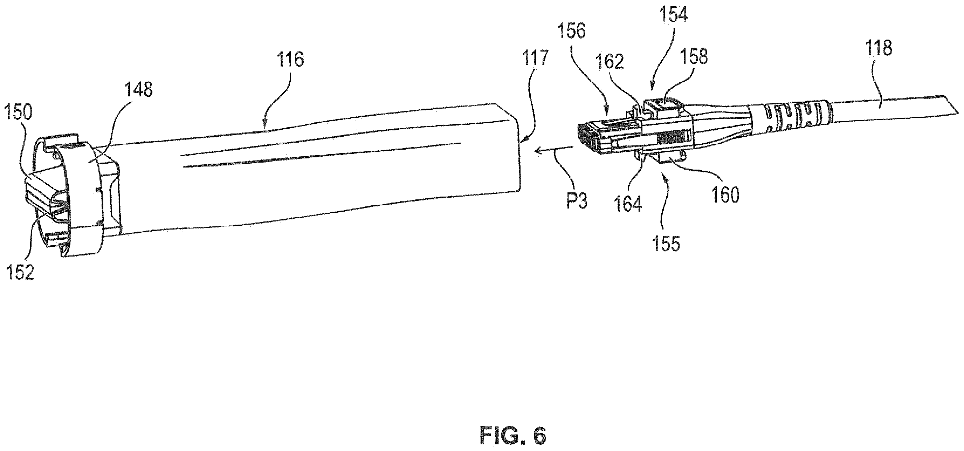

Figur 6 zeigt die Kabelhülle 116 zum sterilen Abschirmen zumindest eines Teils des Endoskopkabels 118. Die Kabelhülle 116 umfasst einen als Steriladapter 148 ausgebildetes Verbindungselement, mit dem der Steckverbinder 156 des Endoskopkabels 118 über Rastverbinder 154, 155 verbindbar ist. Die Rastverbinder 154, 155 sind im vorliegenden Ausführungsbeispiel am Steckverbinder 156 des Endoskopkabels 118 ausgebildet und sind mit zwei Betätigungselementen 158, 160 gekoppelt, mit deren Hilfe die Rastelemente 162, 164 von einer in Figur 6 gezeigten Rastposition in eine Löseposition bewegbar sind, in der die Rastelemente 162, 164 nicht mehr mit den komplementären Eingriffselementen des Steriladapters 148 in Eingriff stehen, so dass der Steckverbinder 156 wieder vom Steriladapter 148 gelöst werden kann.

Zum sterilen Abschirmen des Kabels 118 mit Hilfe der Kabelhülle 116 gegenüber dem sterilen Bereich 110 wird der Steckverbinder 156 des Kabels 118 in das offene Ende 117 der Kabelhülle 116 zusammen mit einem Abschnitt des Kabels 118 eingeführt. Hierzu wird der Steckverbinder 156 in Richtung des Pfeils P3 bewegt, bis der Steckverbinder 156 den Steriladapter 148 kontaktiert und die Rastelemente 162, 164 der Rastverbinder 154, 155 in die zwei komplementäre Eingriffselemente des Steriladapters 148 eingreifen, so dass der Steckverbinder 156 fest mit dem Steriladapter 148 verbunden ist.

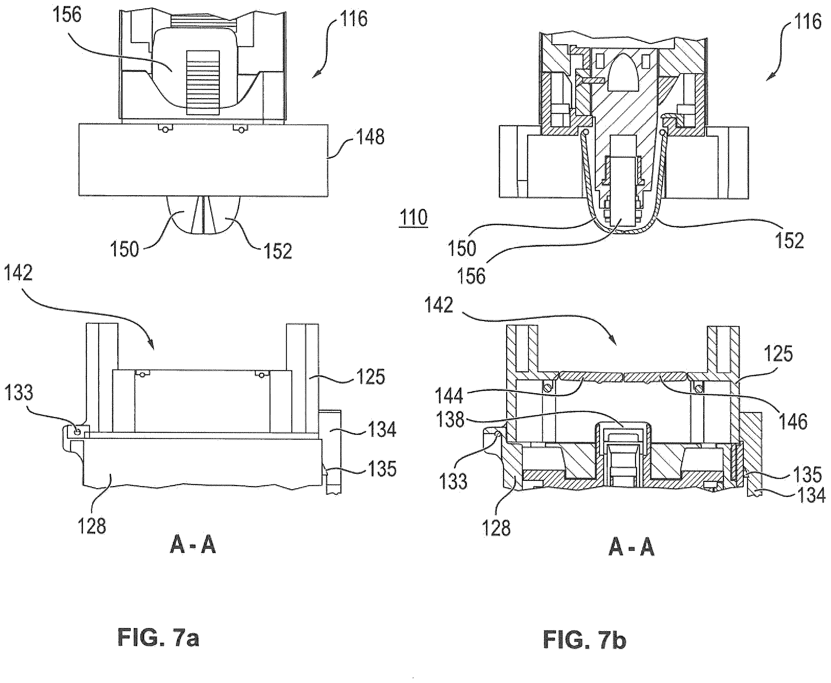

Figur 7a zeigt eine Seitenansicht eines Abschnitts des in der Endoskophülle 112 aufgenommen Endoskops 114 und des mit dem Steriladapter 148 der Kabelhülle 116 verbundenen Steckverbinders 156 des Endoskopkabels 118 in einer ersten Position vor dem Verbinden des Steckverbinders 156 des Endoskopkabels 118 mit dem Steckverbinder 138 des Endoskops 114. Figur 7b zeigt eine Schnittdarstellung der Anordnung nach Figur 7a entlang der in Figur 3 schematisch dargestellten Schnittebene A-A.

Wie in den Figuren 7a und 7b zu sehen ist, sind die Sterilklappen 150, 152 des Steriladapters 148 geschlossen und schirmen den Steckverbinder 156 gegenüber dem sterilen Bereich 110 ab. Des Weiteren sind die Sterilklappen 144, 146 der Sterilschleuse 142 des Verschlusselements 125 der Sterilhülle 112 geschlossen und schirmen den Steckverbinder 138 des Endoskops 114 gegenüber dem sterilen Bereich 110 ab.

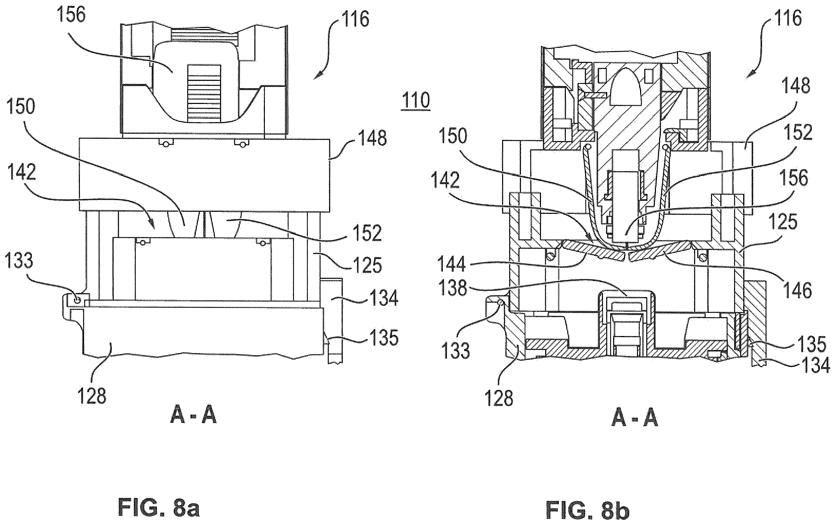

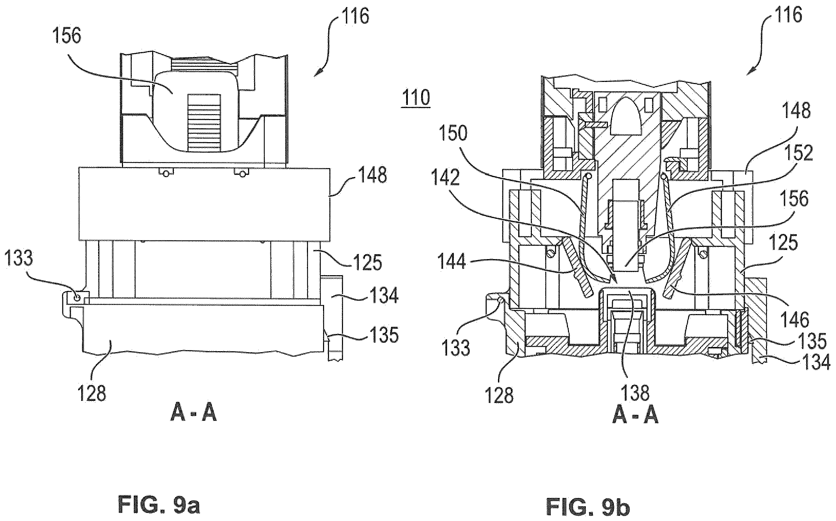

Figur 8a zeigt die Anordnung nach Figur 7a in einer zweiten Position beim Zusammenführen des Steckverbinders 156 des Endoskopkabels 118 mit dem Steckverbinder 138 des Endoskops 114. Figur 8b zeigt eine Schnittdarstellung der Anordnung nach Figur 8a entlang der Schnittebene A-A. Beim Zusammenführen des Steriladapters 148 der Kabelhülle 116 kontaktieren die sterilen Außenseiten der Sterilklappen 150, 152 die sterilen Außenseiten der Sterilklappen 144, 146 der Sterilschleuse 142 und beginnen diese zu öffnen. Zuvor wurden über nicht dargestellte Eingriffselemente Verriegelungselemente zum Verriegeln der Sterilklappen 144, 146 entriegelt, so dass die Sterilklappen 144, 146 von ihrer geschlossenen Position in ihre geöffnete Position bewegt werden können. Sowohl das Verschlusselement 125 der Endoskophülle 112 als auch der Steriladapter 148 der Kabelhülle 116 haben zueinander komplementäre Führungselemente, die so ausgebildet sind, dass eine Verbindung der Steckverbinder 138, 156 nur in der in den Figuren 7a, 7b, 8a, 8b gezeigten Position möglich ist. Durch diese Führungselemente ist ein Zusammenführen der Steckverbinder 138, 156 bei der beschriebenen Ausführungsform nur in einer Position möglich, in der die Steckverbinder 138, 156 exakt gegenüberliegend angeordnet sind, so dass die elektrischen Kontaktelemente und optischen Elemente der Steckverbinder 138, 156 exakt zueinander positioniert sind und hierdurch einfach durch bloßes Zusammenschieben des in der Kabelhülle 116 befindlichen Endoskopkabels 118 und des in der Endoskophülle 112 befindlichen Endoskops 114 verbindbar sind, wie dies weiter in Verbindung mit den Figuren 9a bis 11b noch gezeigt und erläutert wird.

Figur 9a zeigt die Anordnung nach den Figuren 7a und 8a in einer dritten Position, in der der Steckverbinder 156 des Endoskopkabels 118 und der Steckverbinder 138 des Endoskops 114 gegenüber ihren Positionen in den Figuren 7a, 7b, 8a, 8b weiter angenähert worden sind. Wie insbesondere in Figur 9b zu sehen ist, sind die Sterilklappen 144, 146 der Sterilschleuse 142 durch ihren Kontakt mit den Sterilklappen 150, 152 des Steriladapters 148 geöffnet worden. Darüber hinaus sind die Sterilklappen 150, 152 des Steriladapters 148 durch Kontakt mit entsprechenden Eingriffselementen des Verschlusselements 125 und der Zusammenführbewegung von der in den Figuren 8a, 8b gezeigten Position in die in den Figuren 9a, 9b gezeigte Position von ihrer vollständig geschlossenen Position in eine etwas geöffnete Position bewegt worden, die in Figur 9b dargestellt ist. Die Steckverbinder 138, 156 sind weiterhin gegenüberliegend angeordnet und in der in den Figuren 9a, 9b gezeigten Position gegenüber der in den Figuren 8a, 8b gezeigten Position weiter angenähert.

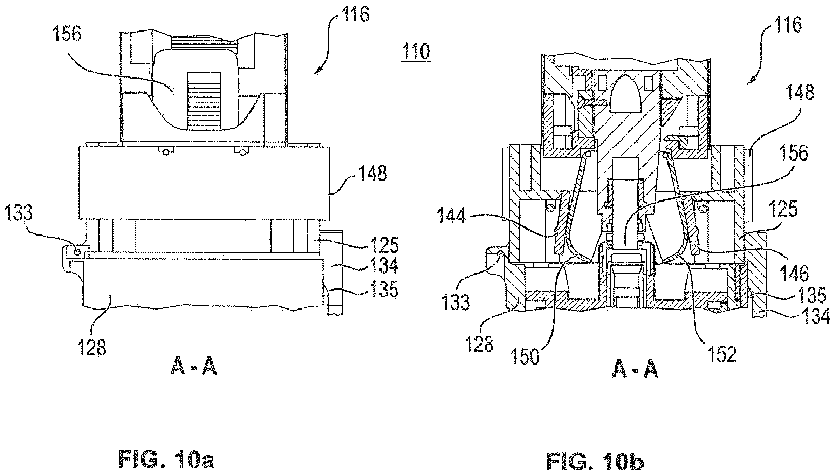

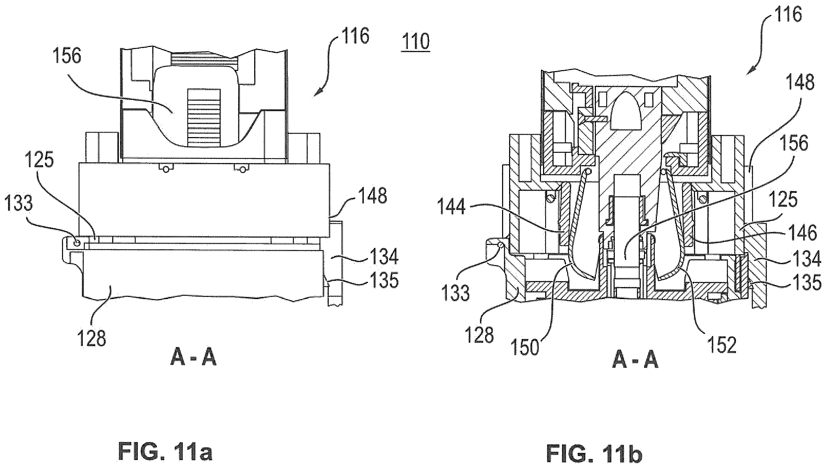

Figur 10a zeigt die Anordnung nach den Figuren 7a, 8a und 9a in einer weiteren Position, in der der Steckverbinder 156 des Endoskopkabels 118 und der Steckverbinder 138 des Endoskops 114 weiter angenähert sind und die optischen Übertragungselemente der Steckverbinder 138, 156 zum Herstellen einer optischen Verbindung zwischen dem Endoskopkabel 118 und dem Endoskop 114 sowie die elektrischen Kontaktelemente derart gegenüberliegend angeordnet sind, dass sich die Steckverbinder 138, 156 bei einer weiteren Bewegung in Richtung des Pfeils P4 direkt kontaktieren, so dass die optischen Übertragungselemente in einer geeigneten Übertragungsposition zueinander angeordnet sind und sich die elektrischen Kontaktelemente kontaktieren, wie dies in den Figuren 11a und 11b gezeigt ist. Bei der in den Figuren 10a und 10b gezeigten Position sind sowohl die Sterilklappen 144, 146 der Sterilschleuse 142 als auch die Sterilklappen 150, 152 des Steriladapters 148 vollständig geöffnet, wobei sich in dieser Position die Gehäuse der Steckverbinder 138, 156 bereits berühren. Bei einer weiteren Bewegung des Steriladapters 148 zusammen mit dem Steckverbinder 156 in Richtung des Pfeils P4 werden die Steckverbinder 138, 156 in einen korrekt verbundenen Zustand gebracht, wie dies in Figur 11a und Figur 11b gezeigt ist. Über die Eingriffselemente zum Öffnen der Sterilklappen 150, 152 werden diese bei einer Bewegung von der in Figur 10a, 10b gezeigten Position in die in Figur 11a, 11b gezeigten Position wieder etwas geringfügig geschlossen, wobei das Schließen der Sterilklappen 150, 152, 144, 146 durch die Federkraft jeweils einer mit einer der Sterilklappen 144, 146, 150, 152 in Eingriff stehenden Feder bewirkt wird. Die Federn werden beim Öffnen der jeweiligen Sterilklappe 144, 146, 150, 152 gespannt und beim Schließen der jeweiligen Sterilklappe 144, 146, 150, 152 entspannt.

Beim Trennen der Steckverbinder 138, 156 werden diese aus ihrer in den Figuren 11a, 11b gezeigten Position in ihre in den Figuren 7a, 7b gezeigte Position bewegt. Dabei erfolgt eine Bewegung des Steriladapters 148 in eine Richtung entgegengesetzt des Pfeils P4, wobei sowohl die Sterilklappen 144, 146 der Sterilschleuse 142 als auch die Sterilklappen 150,152 des Steriladapters 148 automatisch geschlossen und im geschlossenen Zustand verriegelt werden. Somit sind die nicht sterilen Steckverbinder 138, 156 auch nach dem Trennen des Kabels 118 vom Endoskop 114 wieder steril abgedeckt, so dass eine Kontaminierung des sterilen Bereichs 110 wirksam verhindert wird.

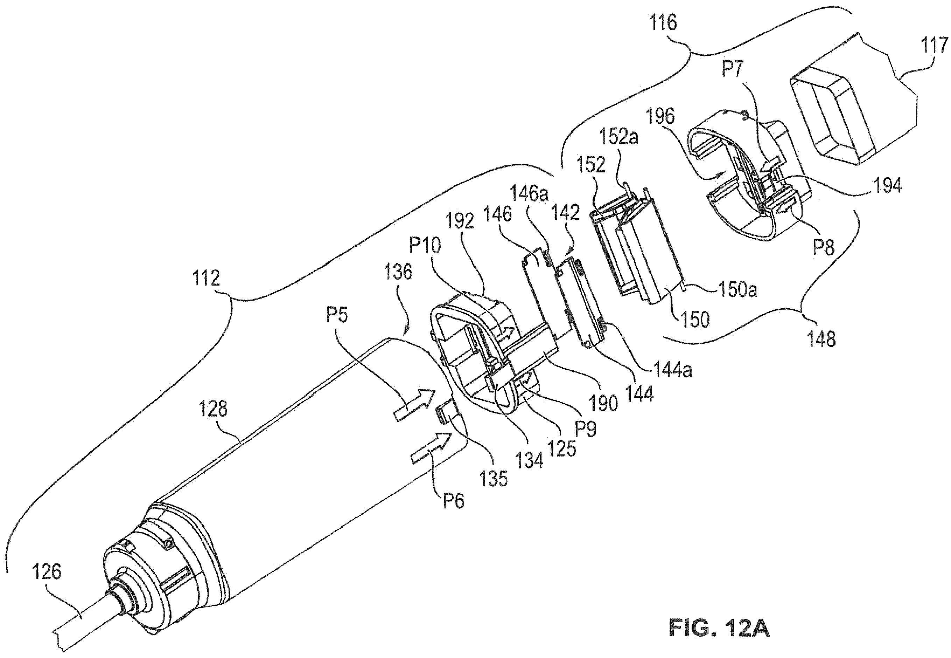

Figur 12a zeigt eine Explosionsdarstellung der Elemente der Endoskophülle 112 und der Elemente der Kabelhülle 116 mit geöffneten Sterilklappen 144, 146, 150, 152. Sowohl an der Mantelfläche des Mittelteils 128 der Endoskophülle 112 als auch an dem Steriladapter 148 und dem Verschlusselement 125 sind von außen sichtbare Pfeile P5 bis P10 vorgesehen, die der Bedienperson einen Hinweis geben, wie die Kabelhülle 118 mit dem Steriladapter 148 mit dem Verschlusselement 125 der Endoskophülle 112 zusammenzuführen zu sind, damit eine Verbindung zwischen dem Endoskopkabel 118 und dem Endoskop 114 hergestellt wird, wie dies zuvor in Verbindung mit den Figuren 7a bis 11b erläutert worden ist. Zur drehbaren Lagerung haben die Sterilklappen 144, 146, 150, 152 haben jeweils Verbindungsstifte 144a, 146a, 150a, 152a, mit deren Hilfe die jeweilige Sterilklappe 144, 146, 150, 152 um die Längsachse des jeweiligen Stifts 144a, 146a, 150a, 152a in entsprechenden Aufnahmen des Verschlusselements 125 bzw. der Sterilschleuse 148 drehbar gelagert sind, so dass sie um die jeweilige Drehachse von einer geschlossenen Position in eine geöffnete Position verschwenkbar sind und umgekehrt. In Figur 12a sind die Sterilklappen 144, 146, 150, 152 in ihrer geöffneten Position dargestellt, in der sie sich befinden, wenn der Steriladapter 148 mit dem Verschlusselement 125 verbunden ist. Das Verschlusselement 125 hat ferner zwei Eingriffselemente 190, 192, die in entsprechenden Aufnahmen 194, 196 des Steriladapters 148 eingreifen, wenn der Steriladapter 148 der Kabelhülle 116 mit dem Verschlusselement 125 der Endoskophülle 112 verbunden ist.

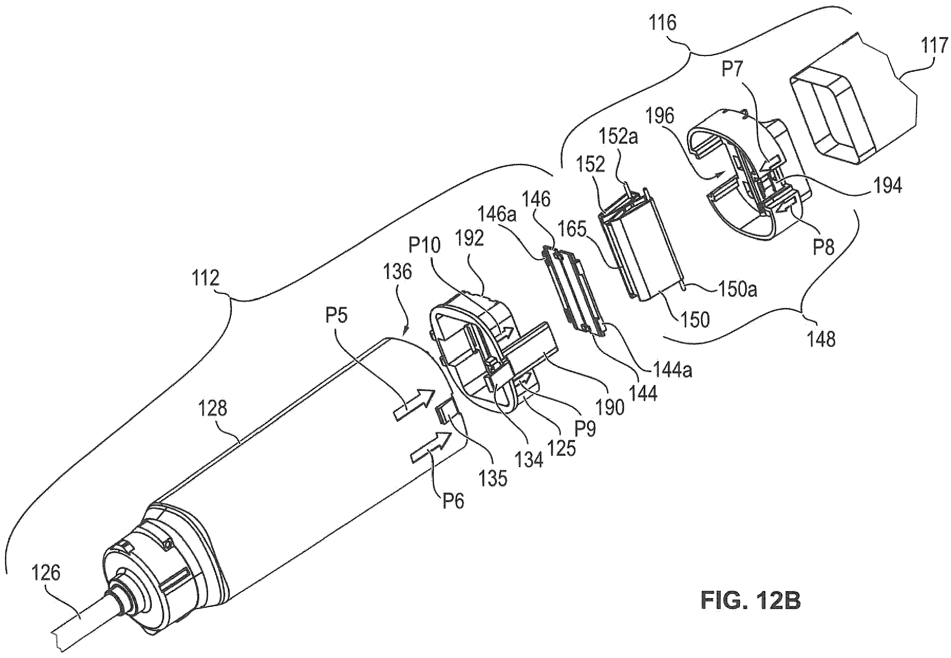

Figur 12b zeigt die Explosionsdarstellung der Figur 12a mit geschlossenen Sterilklappen. In dieser Position sind die Sterilklappen 144, 146, 150, 152 angeordnet, wenn der Steriladapter 148 nicht mit dem Verschlusselement 125 verbunden ist. In dieser geschlossenen Position sind die Sterilklappen 144, 146, 150, 152 mit Hilfe nicht dargestellter Verriegelungselemente verriegelt, so dass die Sterilklappen 144, 146, 150, 152 nicht durch Druck auf die Sterilklappen 144, 146 bzw. durch das Einführen eines Gegenstands in den Öffnungsspalt 165 zwischen den Sterilklappen 150, 152 versehentlich geöffnet werden können.

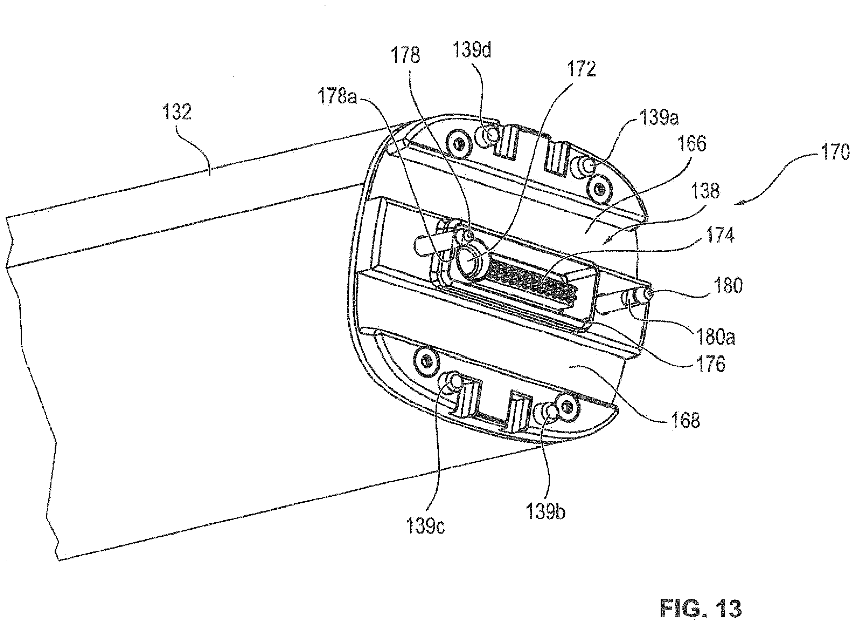

Figur 13 zeigt eine perspektivische Ansicht eines Abschnitts des Endoskops 114 mit dem Steckverbinder 138. Der Steckverbinder 138 ist an der Rückseite des Endoskops 114, d.h. auf der dem stabförmigen Bereich 126 gegenüberliegenden Seite des Endoskops 114, angeordnet. Diese Rückseite ist in Figur 13 mit dem Bezugszeichen 170 bezeichnet. An der Rückseite 170 sind zwei Vertiefungen 166, 168 vorgesehen, die streifenförmig ausgebildet sind und zwischen denen der Steckverbinder 138 des Endoskops 114 angeordnet ist. Im verbundenen Zustand befinden sich die vorderen Abschnitte der Sterilklappen 150, 152 in diesen Vertiefungen 166, 168, wie dies in Figur 11b zu sehen ist. Der Steckverbinder 138 hat einen optischen Übertragungskanal 172 zum Übertragen von Beleuchtungslicht, vom Endoskop 114 detektierten Umgebungslichts und/oder zur Übertragung von optischen Signalen, insbesondere zur Datenübertragung. Darüber hinaus umfasst der Steckverbinder 138 im vorliegenden Ausführungsbeispiel 24 Kontaktelemente, mit denen eins mit dem Bezugszeichen 174 bezeichnet ist. Bei anderen Ausführungsbeispielen können auch mehr oder weniger Kontaktelemente vorgesehen sein, insbesondere können die Kontaktelemente auch eine unterschiedliche Form und/oder Größe haben. Mit Hilfe dieser Kontaktelemente können Signale, Daten und/oder elektrische Energie beispielsweise zur Versorgung von Steuereinheiten, Bilderfassungseinheiten und/oder Beleuchtungseinheiten des Endoskops 114 übertragen werden. Im vorliegenden Ausführungsbeispiel sind die Kontaktelemente 174 und der optische Übertragungskanal 172 in einem gemeinsamen Steckverbindergehäuse 176 angeordnet. Das Gehäuse 176 hat im vorliegenden Ausführungsbeispiel eine längliche Form, wobei die Längsseiten des Gehäuses 176 parallel zu den Längsachsen der Vertiefung 166, 168 ausgerichtet sind. Ferner umfasst der Steckverbinder 138 zwei neben dem Gehäuse 176 angeordnete Führungs- und Verriegelungsstifte 178, 180. Die Führungs- und Verriegelungsstifte 178, 180 haben jeweils mindestens eine Vertiefung 178a, 180a, in die ein komplementäres Rastelement des Steckverbinders 156 des Endoskopkabels 118 eingreifen kann.

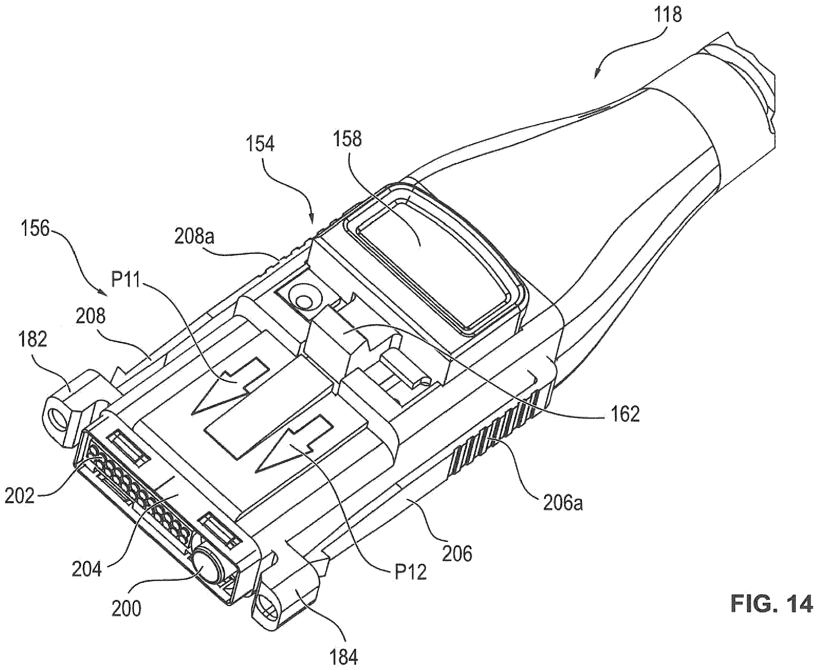

Figur 14 zeigt eine perspektivische Ansicht eines Abschnitts des Endoskopkabels 118 zusammen mit dem am Ende des Endoskopkabels 118angeordneten Steckverbinder 156. Wie bereits in Verbindung mit Figur 6 erläutert, dient der Rastverbinder 154, 155 mit dem Betätigungselementen 158,160 und der Rastelemente 162, 164 zum Verbinden des Steckverbinders 156 im Steriladapter 148. Durch Betätigung der Betätigungselemente 158, 160 kann diese Rastverbindung wieder gelöst und der Steckverbinder 156 vom Steriladapter 148 getrennt werden. Der Steckverbinder 156, der auch als Stecker bezeichnet wird, hat einen optischen dem Übertragungskanal 200, der kompatibel zum optischen Übertragungskanal 142 des Steckverbinders 138 des Endoskops 114 ist. Darüber hinaus hat der Steckverbinder 156 24 elektrische Kontaktelemente, von denen eins mit dem Bezugszeichen 202 bezeichnet ist, die kompatibel zu den elektrischen Kontaktelementen 174 des Steckverbinders 138 sind. Ferner hat der Steckverbinder 156 ein Gehäuse 204, das in das Gehäuse 176 des Steckverbinders 138 des Endoskops 114 einführbar ist. Darüber hinaus hat der Steckverbinder 156 neben den Stirnseiten des länglichen Gehäuses 204 jeweils eine Führungsbuchse 182, 184, durch die im verbundenen Zustand der Steckverbinder 138, 156 die Führungs- und Verriegelungsstifte 178, 180 hindurchgeführt sind, so dass ein Rastelement 206, 208 in jeweils eine Vertiefung 178a, 180a der Führungs- und Verriegelungsstifte 178, 180 eingreift und eine Rastverbindung bildet. Durch Drücken auf die Betätigungsbereiche 206a, 208a der Rastelemente 206, 208 kann eine zwischen den Führungs- und Verriegelungsstiften 178, 180 und den Rastelementen 206, 208 hergestellte Rastverbindung wieder gelöst werden. Die Richtungspfeile P11, P12 geben einem Benutzer die Richtung an, in der der Steckverbinder 156 für eine Verbindung mit dem Steckverbinder 138 zu bewegen ist, um diese zusammenzuführen, bzw. die Richtung an, in der der Steckverbinder 156 zu bewegen ist, um diesen mit dem Steriladapter 148 zu verbinden.

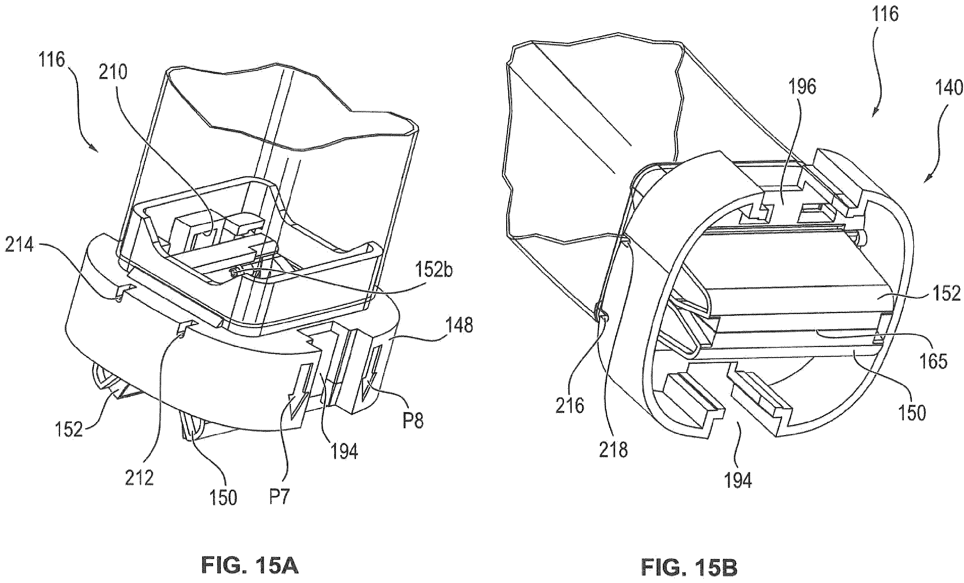

Figur 15a zeigt eine erste perspektivische Ansicht der Kabelhülle 116 mit dem Steriladapter 148 der Kabelhülle 116. Figur 15b zeigt eine zweite perspektivische Ansicht der Kabelhülle der 116 mit dem Steriladapter 148. In den Figuren 15a, 15b sind Aufnahmeöffnungen 212 bis 218 zu sehen, in die die Führungsstifte 150a, 152a der Sterilklappe 150, 152 eingesetzt sind, so dass die Sterilklappen 150, 152 um die Längsachsen der Stifte 150a, 152a verschwenkbar sind. Jeder Sterilklappe 144, 146, 150, 152 ist jeweils eine Feder zugeordnet, die die jeweilige Sterilklappe 144, 146, 150, 152 in ihrer geschlossenen Position hält, wobei die Sterilklappe 144, 146, 150, 152 nur gegen die Federkraft der Feder geöffnet werden kann. Dadurch bewegt die Feder die Sterilklappe 144, 146, 150, 152 in ihre geschlossene Position, wenn die Sterilklappe 144, 146, 150, 152 geöffnet ist. In Figur 15a ist die Feder der Sterilklappe 152 mit dem Bezugszeichen 152b bezeichnet. Die Sterilschleuse 148 hat ferner zwei einander gegenüberliegende Eingriffselemente, von denen in Figur 15a ein Eingriffselement sichtbar und mit dem Bezugszeichen 210 bezeichnet ist. In diese Eingriffselemente 210 greifen die Rastelemente 162, 164 des Steckverbinders 156 ein, wenn der Steckverbinder 156 mit dem Steriladapter 148 verbunden ist.

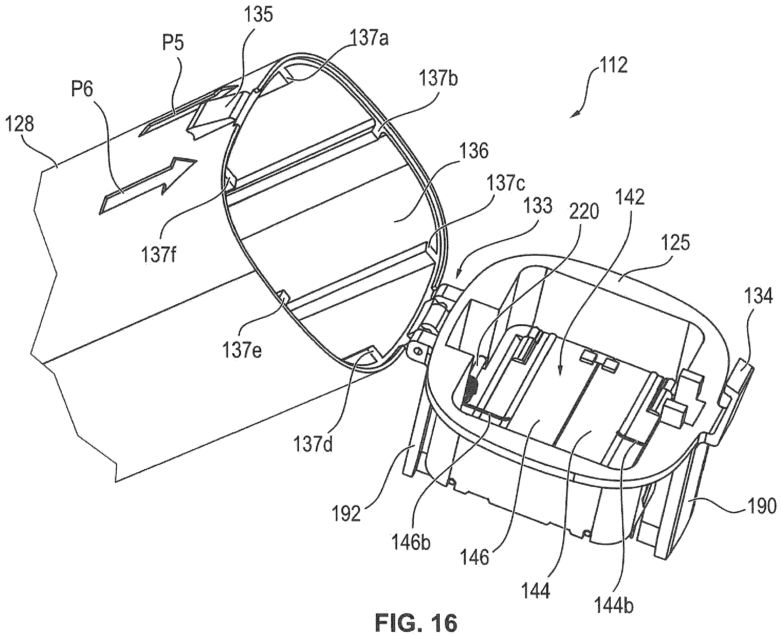

Figur 16 zeigt die Endoskophülle 112 und das Verschlusselement 125 der Endoskophülle in einem geöffneten Zustand. In Figur 16 sind die Federn 144b, 146b, der Sterilklappen 144, 146 dargestellt. Wie bei der Feder 146b zu sehen ist, handelt es sich hierbei um eine Spiralfeder, die um einen Führungsstift 220 herum angeordnet ist, wobei das nicht sichtbare Federende im Eingriff mit dem Verschlusselement 125 steht.

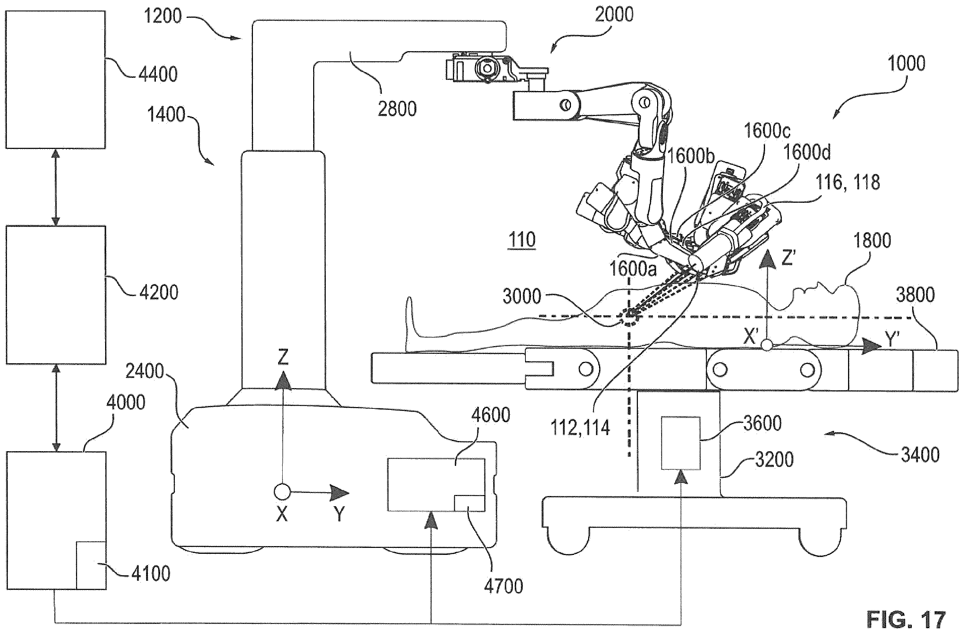

Figur 17 zeigt eine schematische Seitenansicht eines Systems 1000 zur robotergestützten Chirurgie mit einem Manipulator 1200, der ein Stativ 1400 und vier Manipulatorarme 1600a bis 1600d hat. Der Manipulator 1200 wird allgemein auch als Vorrichtung zur robotergestützten Chirurgie bezeichnet. Das System 1000 dient zur Durchführung eines chirurgischen Eingriffs an einem auf einem Operationstisch 3400 positionierten Patienten 1800. Ausgehend von der Anatomie des Patienten 1800 und der durchzuführenden Operation sind die Koordinaten x'z, y'z, z'z eines Zieloperationsgebiets 3000 in einem Patientenkoordinatensystem X', Y', Z' ermittelt und diese Koordinaten x'z, y'z, z'z voreingestellt gespeichert worden. Der Manipulator 1200 hat ein Koordinatensystem X, Y, Z der Vorrichtung 1200, dessen Koordinatenursprung in einem Stativfuß 2400 eines Stativs 1400 des Manipulators 1200 angeordnet ist. Das Stativ 1400 hat einen L-förmigen Stativarm 2800, an dessen vom Stativfuß 2400 entfernten Ende die Manipulatorarme 1600a bis 1600d über einen Stativkopf 2000 verbunden sind.

Der Operationstisch 3400 hat eine Operationstischsäule 3200, in der eine Steuereinheit 3600 des Operationstischs 3400 angeordnet ist und auf der eine mehrere Segmente umfassende Patientenlagerfläche 3800 angeordnet ist. Die Steuereinheit 3600 dient zur Steuerung der Bewegung von Elementen des Operationstischs 3400, insbesondere zur Längenverstellung der Operationstischsäule 3200 und somit zum Verstellen der Höhe der Patientenlagerfläche 3800 und zum Verstellen einzelner Segmente sowie der Neigung und der Kantung der Patientenlagerfläche 3800. Vorzugsweise ist jedoch die Verstellung der Segmente des Operationstischs 3400 während eines operativen Eingriffs mit Hilfe des Manipulators 1200 gesperrt. Das System 1000 umfasst ferner eine Steuereinheit 4600 des Manipulators 1200 sowie eine zentrale Steuereinheit 4000, die über Datenleitungen mit der Steuereinheit 4600 des Manipulators 1200, der Steuereinheit 3600 des Operationstischs 3400 sowie einer Bedienkonsole 4200 mit einer Anzeigeeinheit 4400 verbunden ist. Die Steuereinheit 4000 hat eine Ausgabeeinheit 4100 und die Steuereinheit 4600 hat eine Ausgabeeinheit 4700, durch die jeweils optische und/oder akustische Signale ausgegeben werden können.

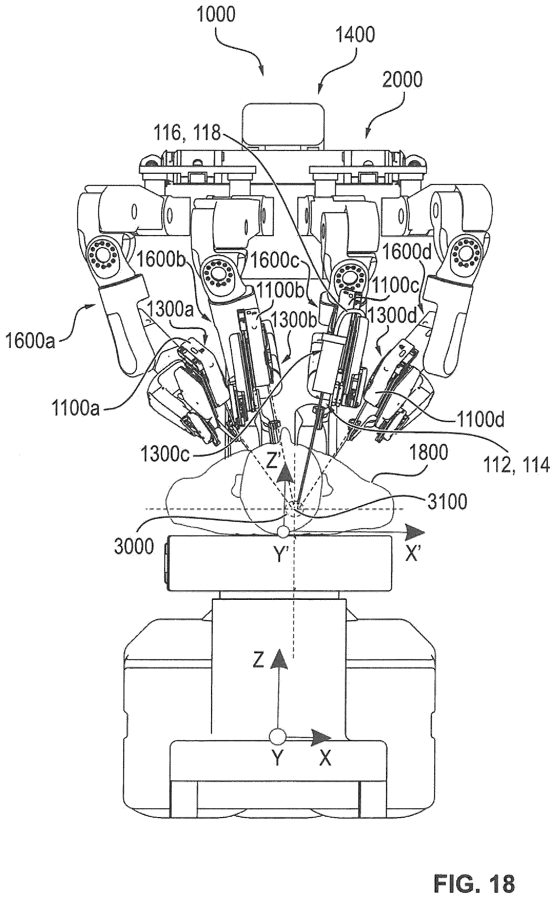

Figur 18 zeigt eine schematische Vorderansicht des Systems 1000 nach Figur 17. Am proximalen Ende der Manipulatorarme 1600a bis 1600d ist jeweils eine Koppeleinheit 1100a bis 1100d angeordnet, mit den jeweils eine Instrumenteneinheit 1300a bis 1300d zum Durchführen des chirurgischen Eingriffs verbunden ist. Die Instrumentenschäfte des jeweiligen chirurgischen Instruments der Instrumenteneinheiten 1300a bis 1300d sind durch Strichlinien angedeutet.

Die Instrumenteneinheit 1300c ist bei der gezeigten Ausführungsform das in der Endoskophülle 112 aufgenommene Stabendoskop 114, das über das in der Kabelhülle 116 aufgenommene Kabel 118 mit der Steuereinheit 4100 verbunden ist. Die anderen Instrumenteneinheiten 1300a, 1300b und 1300d sind im vorliegenden Ausführungsbeispiel Instrumenteneinheiten mit Endeffektoren, insbesondere zum Durchführen des chirurgischen Eingriffs mit Hilfe des Systems 1000. Bei anderen Ausführungsformen können auch zwei Endoskope und zwei chirurgische Instrumente mit den Manipulatorarmen 1600a bis 1600d verbunden sein. Bei anderen Ausführungsformen können auch mehr oder weniger Manipulatorarme 1600a bis 1600d vorgesehen sein oder nur einige der vorhandenen Manipulatorarme 1600a bis 1600d für eine Operation genutzt werden.

### Bezugszeichenliste

- 10,100: Anordnung
- 12, 112: Endoskophülle
- 14,114: Endoskop
- 16, 116: Kabelhülle
- 18, 1118: Endoskopkabel
- 20: Steckverbinder
- 26: stabförmiger Bereich
- 28: Spitze
- 30: optisches Element
- 32, 132: Endoskopkörper
- 34, 36: mechanisches Verbindungselement
- 38: elektrischer Kontakt
- 40: optisches Verbindungselement
- 42, 142: Sterilschleuse
- 44, 46, 144, 146, 50, 52, 150, 152: Sterilklappe
- 48, 148: Steriladapter/Sterilschleuse
- 54, 56: mechanisches Verbindungselement
- 110: steriler Bereich
- 117: offenes Ende
- 125: Verschlusselement
- 126: stabförmiger Bereich
- 127: Spitze
- 128: Mittelteil der Endoskophülle
- 129: vorderer Teil der Endoskophülle
- 130: optisches Element
- 133: Scharnier
- 134: Rastverbinder
- 135: Eingriffselement
- 136: Zuführ- und Entnahmeöffnung
- 137a bis 137e: Führungsstege
- 138: Hybridsteckverbinder
- 139a bis 139d: Distanzstifte
- 154, 155: Rastverbinder
- 156: Steckverbinder
- 158, 160: Betätigungselement
- 162, 164: Rastelement
- 144a, 146a, 150a, 152a: Führungsstifte
- 144b, 146b: Feder
- 165: Öffnungsspalt
- 166, 168: Vertiefung
- 170: Rückseite
- 172: optischer Übertragungskanal
- 174: elektrisches Kontaktelement
- 176: Gehäuse
- 178, 180: Führungs- und Verriegelungsstifte
- 178a, 180a: Vertiefung
- 182, 184: Führungsbuchse
- 190, 192: Rastelement
- 194, 196: Eingriffsöffnungen
- 200: optischer Übertragungskanal
- 202: elektrischer Kontakt
- 204: Gehäuse
- 206, 208: Rastelemente
- 206a, 208a: Betätigungsbereich
- 210: Eingriffselement
- 212, 214, 216, 218: Aufnahmebereich
- 220: Führungsstift
- 144b, 146b: Feder
- P1 bis P12: Richtungspfeile
- 1000: System
- 1200: Manipulator
- 1400: Stativ
- 1600a bis 1600d: Manipulatorarm
- 1800: Patient
- 2000: Stativkopf
- 2400: Stativfuß
- 2800: Stativarm
- 3000: Zieloperationsgebiet
- 3200: Operationstischsäule
- 3400: Operationstisch
- 3600: Steuereinheit des Operationstisches
- 3800: Patientenlagerfläche
- 4000: zentrale Steuereinheit der Vorrichtung
- 4100: Ausgabeeinheit
- 4200: Bedienkonsole
- 4400: Anzeigeeinheit
- 4600: Steuereinheit des Manipulators
- 4700: Ausgabeeinheit
- 1100a bis 1100d: Koppeleinheit
- 1300a bis 1300d: Instrumenteneinheit

## Patentansprüche

1. Anordnung zur sterilen Handhabung eines nicht sterilen Endoskops in einer sterilen Umgebung,
mit einer sterilen Endoskophülle (12, 112) zur Aufnahme des Endoskops (14, 114),
mit einem Kabel (18, 118), das mit dem Endoskop (14, 114) verbindbar ist,
mit einer sterilen Kabelhülle (16, 116) zur Aufnahme zumindest eines Abschnitts des Kabels (18,118),
**dadurch gekennzeichnet, dass**
die Endoskophülle (12, 112) eine erste Sterilschleuse (42, 142) umfasst, die mindestens eine erste Sterilklappe (44, 46, 144, 146) umfasst, die im geschlossenen Zustand das in der Endoskophülle (12, 112) angeordnete Endoskop (14, 114) steril abschirmt,
dass die Kabelhülle (16, 116) eine zweite Sterilschleuse (48, 148) umfasst, die eine zweiten Sterilklappe (50, 52, 150, 152) hat, die in einem geschlossenen Zustand das Kabel (18, 118) und/oder einen am Kabel (18, 118) vorhandenen Steckverbinder (156) steril abschirmt,
dass beim Verbinden des Kabels (18, 118) bzw. des Steckverbinders (156) mit dem Endoskop (14, 114) eine Bewegung der ersten Sterilklappe (44, 46, 144, 146) von dem geschlossenen Zustand in einen geöffneten Zustand und eine Bewegung der zweiten Sterilklappe (50, 52, 150, 152) von dem geschlossenen Zustand in einen geöffneten Zustand erfolgt, so dass eine direkte Kopplung des Kabels (18, 118) mit dem Endoskop (14, 114)oder des mit dem Kabel (18, 118) verbundenen Steckverbinders (156) mit einem komplementären Steckverbinder (138) des Endoskops (14, 114) möglich ist, und
dass beim Trennen des Kabels (18, 118) vom Endoskop (14, 114) eine Bewegung der ersten Sterilklappe (44, 46, 144, 146) von dem geöffneten Zustand in den geschlossenen Zustand und eine Bewegung der zweiten Sterilklappe (50, 52, 150, 152) von dem geöffneten in den geschlossenen Zustand erfolgt, so dass die erste Sterilklappe (44, 46, 144, 146) nach dem Trennen das in der Endoskophülle (12, 112) angeordnete Endoskop (14, 114) steril abschirmt und die zweite Sterilklappe (50, 52, 150, 152) nach dem Trennen das in der Kabelhülle (16, 116) angeordnete Kabel (18, 118) und/oder den Steckverbinder (156) steril abschirmt.

2. Anordnung nach Anspruch 1, **dadurch gekennzeichnet, dass** die zweite Sterilschleuse (48, 148) mit dem Kabel (18, 118) oder mit dem am Kabel (18, 118) vorhandenen Steckverbinder (156) über eine lösbare Rastverbindung (154, 155) verbindbar ist.

3. Anordnung nach einem der vorhergehenden Ansprüche, **dadurch gekennzeichnet, dass** die Endoskophülle (12, 112) eine Öffnung (136) hat, in die das Endoskop (14, 114) einführbar ist und dass die Öffnung (136) der Endoskophülle (12, 112)mit Hilfe eines Verschlusselements (125) steril verschließbar ist, wobei das Verschlusselement (125) die erste Sterilschleuse (42, 142) umfasst.

4. Anordnung nach einem der vorhergehenden Ansprüche, **dadurch gekennzeichnet, dass** das Kabel (18, 118) ein elektrisches Kabel (18, 118) mit mindestens einem elektrischen Leiter, ein optisches Kabel (18, 118), ein Lichtleitkabel (18, 118) oder ein Hybridkabel (18, 118) mit mindestens einem elektrischen Leiter und mindestens einer optischen Faser ist,
dass der Steckverbinder (156) des Kabels (18, 118) die mindestens eine optische Faser des Kabels (18, 118) mit mindestens einem optischen Element des Endoskops (14, 114) koppelt und/oder dass der Steckverbinder (156) die mindestens eine elektrische Leitung des Kabels (18, 118) mit mindestens einem elektrischen Anschluss des Endoskops (14, 114)verbindet.

5. Anordnung nach einem der vorhergehenden Ansprüche, **dadurch gekennzeichnet, dass** das Endoskop (14, 114) einen ersten Steckverbinder (138) hat, dass an mindestens einem Ende des Kabels (18, 118) ein zum ersten Steckverbinder (138) komplementärer zweiter Steckverbinder (156) vorgesehen ist, der mit dem ersten Steckverbinder (138) des Endoskops (14, 114) verbindbar ist und dass mit Hilfe des ersten Steckverbinders (138) und des zweiten Steckverbinders (156) mindestens eine optische Faser des Kabels (18, 118) mit mindestens einem optischen Element des Endoskops (14, 114) koppelbar ist und/oder dass mit Hilfe des ersten Steckverbinders (138) oder des zweiten Steckverbinders (156) mindestens eine elektrische Leitung des Kabels (18, 118) mit mindestens einem elektrischen Anschluss des Endoskops (14, 114) verbindbar ist.

6. Anordnung nach Anspruch 5, **dadurch gekennzeichnet, dass** beim Verbinden des zweiten Steckverbinders (156) des Kabels (18, 118) mit dem ersten Steckverbinder (138) des Endoskops (14, 114) zwischen dem ersten Steckverbinder (138) und dem zweiten Steckverbinder (156) eine lösbare Rastverbindung erfolgt und
dass zum Trennen des zweiten Steckverbinders (156) des Kabels (18, 118) von dem ersten Steckverbinder (138) des Endoskops (14, 114) zumindest die Rastverbindung zu lösen ist.

7. Anordnung nach einem der vorhergehenden Ansprüche, **dadurch gekennzeichnet, dass** die erste Sterilschleuse (42, 142) bei der Aufnahme des Endoskops (14, 114) in der Endoskophülle (12,112) eine definierte Lage zu einer Koppelschnittstelle (138) des Endoskops (14, 114) hat, wobei die Koppelschnittstelle (138) des Endoskops (14, 114) mindestens einen elektrischen Anschluss des Endoskops (14, 114) oder ein optisches Element umfasst.

8. Anordnung nach einem der vorhergehenden Ansprüche, **dadurch gekennzeichnet, dass** die zweite Sterilschleuse (48, 148)einen ersten Verbindungsbereich (210) hat, mit dem das Kabel (18, 118) und/oder der Steckverbinder (156) verbindbar ist, und dass die zweite Sterilschleuse (48, 148) einen zweiten Verbindungsbereich hat, mit dem ein komplementärer Verbindungsbereich der ersten Sterilschleuse (42, 142) verbindbar ist,
wobei der erste Verbindungsbereich (210) der zweiten Sterilschleuse (48, 148) und der zweite Verbindungsbereich der zweiten Sterilschleuse (48, 148) vorzugsweise auf einander abgewandten Seiten der zweiten Sterilschleuse (48, 148) angeordnet sind.

9. Anordnung nach Anspruch 8, **dadurch gekennzeichnet, dass** der erste Verbindungsbereich (210) der zweiten Sterilschleuse (48, 148) über eine erste lösbare Rastverbindung mit dem Kabel (18, 118) und/oder dem am Ende des Kabels (18, 118) vorgesehenen Steckverbinder (156) verbindbar ist, und dass der zweite Verbindungsbereich der zweiten Sterilschleuse (48, 148) über eine zweite lösbare Rastverbindung mit dem komplementären Verbindungsbereich der ersten Sterilschleuse (42, 142) verbindbar ist.

10. Anordnung nach einem der vorhergehenden Ansprüche, **dadurch gekennzeichnet, dass** die erste Sterilklappe (44, 46, 144, 146) beim Verbinden der ersten Sterilschleuse (42, 142) mit der zweiten Sterilschleuse (48, 148) automatisch öffnet und dass die zweite Sterilklappe (50, 52, 150, 152) beim Verbinden der ersten Sterilschleuse (42, 142) mit der zweiten Sterilschleuse (48, 148) automatisch öffnet, dass die erste Sterilklappe (44, 46, 144, 146) beim Trennen der ersten Sterilschleuse (42, 142) von der zweiten Sterilschleuse (48, 148) automatisch schließt und dass die zweite Sterilklappe (50, 52, 150, 152) beim Trennen der ersten Sterilschleuse (42, 142) von der zweiten Sterilschleuse (48, 148) automatisch schließt.

11. Anordnung nach Anspruch 10, **dadurch gekennzeichnet, dass** beim Verbinden der ersten Sterilschleuse (42, 142) mit der zweiten Sterilschleuse (48, 148) eine automatisch Entriegelung der ersten Sterilklappe (44, 46, 144, 146) erfolgt,
dass beim Verbinden der ersten Sterilschleuse (42, 142) mit der zweiten Sterilschleuse (48, 148) eine automatische Entriegelung der zweiten Sterilklappe (50, 52, 150, 152) erfolgt,
dass beim Trennen der ersten Sterilschleuse (42, 142) von der zweiten Sterilschleuse (48, 148) eine automatische Verriegelung der ersten Sterilklappe (44, 46, 144, 146) erfolgt und
dass beim Trennen der ersten Sterilschleuse (42, 142) von der zweiten Sterilschleuse (48, 148) eine automatische Verriegelung der zweiten Sterilklappe (50, 52, 150, 152) erfolgt.

12. Anordnung nach einem der vorhergehenden Ansprüche, **dadurch gekennzeichnet, dass** eine sterile Außenseite der ersten Sterilklappe (44, 46, 144, 146) beim Verbinden der ersten Sterilschleuse (42, 142) mit der zweiten Sterilschleuse (48, 148) an der sterilen Außenseite der zweiten Sterilklappe (50, 52, 150, 152) gegenüberliegend angeordnet ist, wenn sowohl die erste Sterilklappe (44, 46, 144, 146) als auch die zweite Sterilklappe (50, 52, 150, 152) geöffnet sind, wobei die Außenseite der ersten Sterilklappe (44, 46, 144, 146) und die Außenseite der zweiten Sterilklappe (50, 52, 150, 152) im geöffneten Zustand einander zugewandt sind.

13. Anordnung nach einem der vorhergehenden Ansprüche, **dadurch gekennzeichnet, dass** ein Abschnitt der Endoskophülle (16, 116) einen Schaft (126) eines als Stabendoskop ausgebildeten Endoskops (14, 114) aufzunehmen und vorzugsweise zu umschließen, wobei die Endoskophülle (12, 112) ein optisches Element (30, 130) umfasst, das dem Schaftende (28, 128)des Endoskops (14, 114) gegenüberliegend angeordnet ist, wenn das Endoskop (14, 114) in der Endoskophülle (12, 112) aufgenommen ist.

14. System zur robotergestützten Chirurgie, insbesondere zu einer telerobotergestützten Prozedur innerhalb eines sterilen Bereichs (110),
mit mindestens einer Anordnung (10, 100) nach einem der vorhergehenden Ansprüche,
mit mindestens einem Manipulator (1200), der einen Manipulatorarm (1600a bis 1600d) hat,
mit mindestens einer Eingabeeinrichtung (4200) zur Eingabe mindestens eines Eingabekommandos,
**dadurch gekennzeichnet, dass** das in der Endoskophülle (12,112) aufgenommene Endoskop (14, 114) mit dem Manipulatorarm (1600a bis 1600d) verbunden ist,
dass das System (1000) eine Steuereinheit (4000) umfasst, die den Manipulatorarm (1600a bis 1600d) und das mit dem Manipulatorarm (1600a bis 1600d) verbundene Endoskop (14, 114) abhängig von dem Eingabekommando mit Hilfe mindestens einer Antriebseinheit positioniert.

15. Anordnung zum Herstellen und Lösen einer Steckverbindung zwischen einem ersten nicht sterilen Steckverbinder (138) und einem zum ersten Steckverbinder (138) komplementären zweiten nicht sterilen Steckverbinder (156) in einer sterilen Umgebung (110),
mit einer ersten sterilen Hülle (112) zur Aufnahme zumindest des ersten Steckverbinders (138),
mit einer zweiten sterilen Hülle (116) zur Aufnahme zumindest des zweiten Steckverbinders (156),
**dadurch gekennzeichnet, dass**
die erste Hülle (112) eine erste Sterilschleuse (142) umfasst, die mindestens eine erste Sterilklappe (144, 146) hat, die im geschlossenen Zustand den ersten Steckverbinder (138) steril abschirmt,
die zweite Hülle (116) eine zweite Sterilschleuse (148) umfasst, die mindestens eine zweite Sterilklappe (150, 152) hat, die im geschlossenen Zustand den zweiten Steckverbinder (156) steril abschirmt,
dass beim Verbinden des ersten Steckverbinders (138) mit dem zweiten Steckverbinder (156) eine Bewegung der ersten Sterilklappe (144, 146) von dem geschlossenen Zustand in einen geöffneten Zustand und eine Bewegung der zweiten Sterilklappe (150, 152) von dem geschlossenen Zustand in einen geöffneten Zustand erfolgt, so dass eine direkte Kopplung des ersten Steckverbinders (138) mit dem zweiten Steckverbinder (156) möglich ist, und
dass beim Trennen des ersten Steckverbinders (138) vom zweiten Steckverbinder (156) eine Bewegung der ersten Sterilklappe (144, 146) von dem geöffneten Zustand in den geschlossenen Zustand und eine Bewegung der zweiten Sterilklappe (150, 152) von dem geöffneten Zustand in den geschlossenen Zustand erfolgt, so dass die erste Sterilklappe (144, 146) nach dem Trennen zumindest den ersten Steckverbinder (138) steril abschirmt und die zweite Sterilklappe (150, 152) nach dem Trennen den zweiten Steckverbinder (156) steril abschirmt.

16. Anordnung nach einem der vorhergehenden Ansprüche, **dadurch gekennzeichnet, dass** der erste Steckverbinder (138) mindestens ein erstes elektrisches Kontaktelement (174) zum Herstellen einer elektrischen Verbindung oder mindestens ein erstes optisches Element (172) zur Herstellen einer optischen Verbindung hat,
dass der zweite Steckverbinder (156) mindestens ein zweites elektrisches Kontaktelement (202) zum Herstellen einer elektrischen Verbindung mit dem ersten elektrischen Kontaktelement (174) oder mindestens ein zweites optisches Element (200) zur Herstellen einer optischen Verbindung mit dem ersten optischen Element (172) hat,
dass das mindestens eine erste elektrische Kontaktelement (174) und das mindestens eine zweite elektrische Kontaktelement (202) und/oder dass das mindestens eine erste optische Element (172) und das mindestens eine zweite optische Element (200) derart angeordnet und ausgebildet sind, dass bei einer Verbindung zwischen dem ersten Steckverbinder (138) und dem zweiten Steckverbinder (156) eine elektrische Verbindung zwischen dem ersten elektrischen Kontaktelement (174) und dem zweiten elektrischen Kontaktelement (202) oder eine optische Verbindung zwischen dem ersten optischen Element (172) und dem zweiten optischen Element (200) erfolgt.

17. Anordnung nach einem der vorhergehenden Ansprüche, **dadurch gekennzeichnet, dass** der erste Steckverbinder (138) und der zweite Steckverbinder (156) derart symmetrisch ausgebildet sind, dass die elektrische Verbindung zwischen dem ersten elektrischen Kontaktelement (174) und dem zweiten elektrischen Kontaktelement (202) und/oder die optische Verbindung zwischen dem ersten optischen Element (172) und dem zweiten optischen Element (200) sowohl beim Zusammenführen der Steckverbinder (138, 156) in einer ersten Position und beim Zusammenführen der Steckverbinder (138, 156) in einer zweiten Position möglich ist, bei der einer der Steckverbinder (138, 156) um eine senkrecht zur Kontaktebene der Steckverbinder (138, 156) verlaufende Achse um 180° gedreht worden ist.

## Claims

1. An arrangement for the sterile handling of a non-sterile endoscope in a sterile environment,
with a sterile endoscope sheath (12, 112) for accommodating the endoscope (14, 114),
with a cable (18, 118) that is connectable to the endoscope (14, 114),
with a sterile cable sheath (16, 116) for accommodating at least a portion of the cable (18, 118),
**characterized in that**
the endoscope sheath (12, 112) comprises a first sterile lock (42, 142) comprising at least one first sterile flap (44, 46, 144, 146) which in the closed state shields the endoscope (14, 114) arranged in the endoscope sheath (12, 112) in a sterile manner,
that the cable sheath (16, 116) comprises a second sterile lock (48, 148) having a second sterile flap (50, 52, 150, 152) which in a closed state shields the cable (18, 118) and/or a plug connector (156) present on the cable (18, 118) in a sterile manner,
that when the cable (18, 118) or the plug connector (156) is connected to the endoscope (14, 114) a movement of the first sterile flap (44, 46, 144, 146) from the closed state into an open state and a movement of the second sterile flap (50, 52, 150, 152) from the closed state into an open state takes place so that a direct coupling of the cable (18, 118) to the endoscope (14, 114) or of the plug connector (156) connected to the cable (18, 118) to a complementary plug connector (138) of the endoscope (14, 114) is possible, and
that when the cable (18, 118) is separated from the endoscope (14, 114) a movement of the first sterile flap (44, 46, 144, 146) from the open state into the closed state and a movement of the second sterile flap (50, 52, 150, 152) from the open into the closed state takes place so that the first sterile flap (44, 46, 144, 146) shields the endoscope (14, 114) arranged in the endoscope sheath (12, 112) in a sterile manner after separation and the second sterile flap (50, 52, 150, 152) shields the cable (18, 118) arranged in the cable sheath (16, 116) and/or the plug connector (156) in a sterile manner after separation.

2. The arrangement according to claim 1, **characterized in that** the second sterile lock (48, 148) is connectable to the cable (18, 118) or to the plug connector (156) present on the cable (18, 118) via a disconnectable snap-in connection (154, 155).

3. The arrangement according to one of the preceding claims, characterized that the endoscope sheath (12, 112) has an opening (136) into which the endoscope (14, 114) is insertable, and that the opening (136) of the endoscope sheath (12, 112) is closable in a sterile manner with the aid of a closing element (125), the closing element (125) comprising the first sterile lock (42, 142).

4. The arrangement according to one of the preceding claims, **characterized in that** the cable (18, 118) is an electric cable (18, 118) with at least one electric conductor, an optical cable (18, 118), a light guide cable (18, 118) or a hybrid cable (18, 118) with at least one electric conductor and at least one optical fiber,
that the plug connector (156) of the cable (18, 118) couples the at least one optical fiber of the cable (18, 118) with at least one optical element of the endoscope (14, 114) and/or that the plug connector (156) connects the at least one electric conductor of the cable (18, 118) to the at least one electric connection of the endoscope (14, 114).

5. The arrangement according to one of the preceding claims, **characterized in that** the endoscope (14, 114) has a first plug connector (138), that at at least one end of the cable (18, 118) a second plug connector (156) complementary to the first plug connector (138) is provided, which is connectable to the first plug connector (138) of the endoscope (14, 114), and that with the aid of the first plug connector (138) and of the second plug connector (156) at least one optical fiber of the cable (18, 118) is couplable with at least one optical element of the endoscope (14, 114) and/or that with the aid of the first plug connector (138) or the second plug connector (156) at least one electric conductor of the cable (18, 118) is connectable to at least one electric connection of the endoscope (14, 114).

6. The arrangement according to claim 5, **characterized in that** when the second plug connector (156) of the cable (18, 118) is connected to the first plug connector (138) of the endoscope (14, 114) a disconnectable snap-in connection is established between the first plug connector (138) and the second plug connector (156), and
that for separating the second plug connector (156) of the cable (18, 118) from the first plug connector (138) of the endoscope (14, 114) at least the snap-in connection has to be disconnected.

7. The arrangement according to one of the preceding claims, **characterized in that** the first sterile lock (42, 142) has a defined position relative to a coupling interface (138) of the endoscope (14, 114) when the endoscope (14, 114) is accommodated in the endoscope sheath (12, 112), wherein the coupling interface (138) of the endoscope (14, 114) comprises at least one electric connection of the endoscope (14, 114) or an optical element.

8. The arrangement according to one of the preceding claims, **characterized in that** the second sterile lock (48, 148) has a first connecting area (210) to which the cable (18, 118) and/or the plug connector (156) is connectable, and that the second sterile lock (48, 148) has a second connecting area to which a complementary connecting area of the first sterile lock (42, 142) is connectable,
wherein the first connecting area (210) of the second sterile lock (48, 148) and the second connecting area of the second sterile lock (48, 148) are preferably arranged on opposing sides of the second sterile lock (48, 148).

9. The arrangement according to claim 8, **characterized in that** the first connecting area (210) of the second sterile lock (48, 148) is connectable via a first disconnectable snap-in connection to the cable (18, 118) and/or to the plug connector (156) provided at the end of the cable (18, 118), and that the second connecting area of the second sterile lock (48, 148) is connectable via a second disconnectable snap-in connection to the complementary connecting area of the first sterile lock (42, 142).

10. The arrangement according to one of the preceding claims, **characterized in that** the first sterile flap (44, 46, 144, 146) automatically opens when the first sterile lock (42, 142) is connected to the second sterile lock (48, 148), and that the second sterile lock (50, 52, 150, 152) automatically opens when the first sterile lock (42, 142) is connected to the second sterile lock (48, 148), that the first sterile flap (44, 46, 144, 146) automatically closes when the first sterile lock (42, 142) is separated from the second sterile lock (48, 148), and that the second sterile flap (50, 152, 150, 152) automatically closes when the first sterile lock (42, 142) is separated from the second sterile lock (48, 148).

11. The arrangement according to claim 10, **characterized in that** when the first sterile lock (42, 142) is connected to the second sterile lock (48, 148) an automatic unlocking of the first sterile flap (44, 46, 144, 146) is performed,
that when the first sterile lock (42, 142) is connected to the second sterile lock (48, 148) an automatic unlocking of the second sterile flap (50, 52, 150, 152) is performed,
that when the first sterile lock (42, 142) is separated from the second sterile lock (48, 148) an automatic locking of the first sterile flap (44, 46, 144, 146) is performed, and
that when the first sterile lock (42, 142) is separated from the second sterile lock (48, 148) an automatic locking of the second sterile flap (50, 52, 150, 152) is performed.

12. The arrangement according to one of the preceding claims, **characterized in that** a sterile outside of the first sterile flap (44, 46, 144, 146) is arranged opposite to the sterile outside of the second sterile flap (50, 52, 150, 152) when the first sterile lock (42, 142) is connected to the second sterile lock (48, 148), when both the first sterile flap (44, 46, 144, 146) and the second sterile flap (50, 52, 150, 152) are open, wherein the outside of the first sterile flap (44, 46, 144, 146) and the outside of the second sterile flap (50, 52, 150, 152) face each other in the open state.

13. The arrangement according to one of the preceding claims, **characterized in that** a portion of the endoscope sheath (16, 116) accommodates and preferably surrounds a shaft (126) of an endoscope (14, 114) formed as a rod endoscope, wherein the endoscope sheath (12, 112) comprises an optical element (30, 130) that is arranged opposite to the shaft end (28, 128) of the endoscope (14, 114) when the endoscope (14, 114) is accommodated in the endoscope sheath (12, 112).

14. A system for the robot-assisted surgery, in particular for a telerobot-assisted procedure within a sterile area (110),
with at least one arrangement (10, 100) according to one of the preceding claims,
with at least one manipulator (1200) having a manipulator arm (1600a to 1600d),
with at least one input device (4200) for the input of at least one input command,
**characterized in that** the endoscope (14, 114) accommodated in the endoscope sheath (12, 112) is connected to the manipulator arm (1600a to 1600d),
that the system (1000) comprises a control unit (4000) that positions the manipulator arm (1600a to 1600d) and the endoscope (14, 114) connected to the manipulator arm (1600a to 1600d) dependent on the input command with the aid of at least one drive unit.

15. An arrangement for establishing and disconnecting a plug connection between a first non-sterile plug connector (138) and a second non-sterile plug connector (156) complementary to the first plug connector (138) in a sterile surrounding (110),
with a first sterile sheath (112) for accommodating at least the first plug connector (138),
with a second sterile sheath (116) for accommodating at least the second plug connector (156),
**characterized in that**
the first sheath (112) comprises a first sterile lock (142) which has at least one first sterile flap (144, 146) which in the closed state shields the first plug connector (138) in a sterile manner,
the second sheath (116) comprises a second sterile lock (148) having at least one second sterile flap (150, 152) which in the closed state shields the second plug connector (156) in a sterile manner,
that when the first plug connector (138) is connected to the second plug connector (156) a movement of the first sterile flap (144, 146) from the closed state into an open state and a movement of the second sterile flap (150, 152) from the closed state into an open state takes place so that a direct coupling of the first plug connector (138) to the second plug connector (156) is possible, and
that when the first plug connector (138) is separated from the second plug connector (156) a movement of the first sterile flap (144, 146) from the open state into the closed state and a movement of the second sterile flap (150, 152) from the open state into the closed state takes place so that the first sterile flap (144, 146) shields at least the first plug connector (138) in a sterile manner after separation and the second sterile flap (150, 152) shields the second plug connector (156) in a sterile manner after separation.

16. The arrangement according to one of the preceding claims, **characterized in that** the first plug connector (138) has at least one first electric contact element (174) for establishing an electric connection or at least one first optical element (172) for establishing an optical connection,
that the second plug connector (156) has at least one second electric contact element (202) for establishing an electric connection with the first electric contact element (174) or at least one second optical element (200) for establishing an optical connection with the first optical element (172),
that the at least one first electric contact element (174) and the at least one second electric contact element (202) and/or that the at least one first optical element (172) and the at least one second optical element (200) are arranged and formed such that when the first plug connector (138) is connected to the second plug connector (156) an electrical connection between the first electric contact element (174) and the second electric contact element (202) or an optical connection between the first optical element (172) and the second optical element (200) is established.

17. The arrangement according to one of the preceding claims, **characterized in that** the first plug connector (138) and the second plug connector (156) are symmetrically formed such that the electrical connection between the first electric contact element (174) and the second electric contact element (202) and/or the optical connection between the first optical element (172) and the second optical element (200) is possible both when bringing together the plug connectors (138, 156) in a first position and when bringing together the plug connectors (138, 156) in a second position, in which one of the plug connectors (138, 156) has been rotated by 180° about an axis extending perpendicular to the contact plane of the plug connectors (138, 156).

## Revendications

1. Ensemble pour la manipulation stérile d'un endoscope non stérile dans un environnement stérile,
avec une enveloppe d'endoscope (12, 112) stérile destinée à loger l'endoscope (14, 114),
avec un câble (18, 118), qui peut être relié à l'endoscope (14, 114),
avec une enveloppe de câble (16, 116) stérile destinée à loger au moins une partie du câble (18, 118),
**caractérisé en ce que**
l'enveloppe d'endoscope (12, 112) comprend un premier sas stérile (42, 142), qui comprend au moins un premier clapet stérile (44, 46, 144, 146), qui, dans l'état fermé, protège de manière stérile l'endoscope (14, 114) disposé dans l'enveloppe d'endoscope (12, 112),
**que** l'enveloppe de câble (16, 116) comprend un deuxième sas stérile (48, 148), qui présente un deuxième clapet stérile (50, 52, 150, 152), qui, dans un état fermé, protège de manière stérile le câble (18, 118) et/ou un connecteur électrique (156) présent sur le câble (18, 118) ,
**que**, lors de la liaison du câble (18, 118) ou du connecteur électrique (156) à l'endoscope (14, 114), un déplacement du premier clapet stérile (44, 46, 144, 146) de l'état fermé à un état ouvert et un déplacement du deuxième clapet stérile (50, 52, 150, 152) de l'état fermé à un état ouvert s'effectuent, de sorte qu'un couplage direct du câble (18, 118) à l'endoscope (14, 114) ou du connecteur électrique (156) relié au câble (18, 118) à un connecteur électrique (138) complémentaire de l'endoscope (14, 114) est possible, et
**que**, lors de la séparation du câble (18, 118) de l'endoscope (14, 114), un déplacement du premier clapet stérile (44, 46, 144, 146) de l'état ouvert à l'état fermé et un déplacement du deuxième clapet stérile (50, 52, 150, 152) de l'état ouvert à l'état fermé s'effectuent, de sorte que le premier clapet stérile (44, 46, 144, 146), après la séparation, protège de manière stérile l'endoscope (14, 114) disposé dans l'enveloppe d'endoscope (12, 112) et le deuxième clapet stérile (50, 52, 150, 152), après la séparation, protège le câble (18, 118) disposé dans l'enveloppe de câble (16, 116) et/ou le connecteur électrique (156).

2. Ensemble selon la revendication 1, **caractérisé en ce que** le deuxième sas stérile (48, 148) peut être relié au câble (18, 118) ou au connecteur électrique (156) présent sur le câble (18, 118) par l'intermédiaire d'une liaison par encliquetage (154, 155) libérable.

3. Ensemble selon l'une quelconque des revendications précédentes, **caractérisé en ce que** l'enveloppe d'endoscope (12, 112) présente une ouverture (136) dans laquelle l'endoscope (14, 114) peut être introduit et que l'ouverture (136) de l'enveloppe d'endoscope (12, 112) peut être fermée de manière stérile à l'aide d'un élément de fermeture (125), dans lequel l'élément de fermeture (125) comprend le premier sas stérile (42, 142).

4. Ensemble selon l'une quelconque des revendications précédentes, **caractérisé en ce que** le câble (18, 118) est un câble électrique (18, 118) avec au moins un conducteur électrique, un câble optique (18, 118), un câble à fibre optique (18, 118) ou un câble hybride (18, 118) avec au moins un fil électrique et au moins une fibre optique,
**que** le connecteur électrique (156) du câble (18, 118) relie l'au moins une fibre optique du câble (18, 118) à au moins un élément optique de l'endoscope (14, 114) et/ou que le connecteur électrique (156) relie l'au moins un fil électrique du câble (18, 118) à au moins un raccord électrique de l'endoscope (14, 114).

5. Ensemble selon l'une quelconque des revendications précédentes, **caractérisé en ce que** l'endoscope (14, 114) présente un premier connecteur électrique (138), qu'un deuxième connecteur électrique (156) complémentaire du premier connecteur électrique (138), qui peut être relié au premier connecteur électrique (138) de l'endoscope (14, 114), est prévu à au moins une extrémité du câble (18, 118), et que l'au moins une fibre optique du câble (18, 118) peut être couplée à au moins un élément optique de l'endoscope (14, 114) à l'aide du premier connecteur électrique (138) et du deuxième connecteur électrique (156) et/ou qu'au moins un fil électrique du câble (18, 118) peut être relié à au moins un raccord électrique de l'endoscope (14, 114) à l'aide du premier connecteur électrique (138) ou du deuxième connecteur électrique (156).

6. Ensemble selon la revendication 5, **caractérisé en ce que**, lors de la liaison du deuxième connecteur électrique (156) du câble (18, 118) au premier connecteur électrique (138) de l'endoscope (14, 114), une liaison par encliquetage libérable s'effectue entre le premier connecteur électrique (138) et le deuxième connecteur électrique (156) et
**que**, pour la séparation du deuxième connecteur électrique (156) du câble (18, 118) du premier connecteur électrique (138) de l'endoscope (14, 114), au moins la liaison par encliquetage doit être libérée.

7. Ensemble selon l'une quelconque des revendications précédentes, **caractérisé en ce que** le premier sas stérile (42, 142), lors du logement de l'endoscope (14, 114) dans l'enveloppe d'endoscope (12, 112), présente une position définie par rapport à une interface de couplage (138) de l'endoscope (14, 114), dans lequel l'interface de couplage (138) de l'endoscope (14, 114) comprend au moins un raccord électrique de l'endoscope (14, 114) ou un élément optique.

8. Ensemble selon l'une quelconque des revendications précédentes, **caractérisé en ce que** le deuxième sas stérile (48, 148) présente une première zone de liaison (210), à laquelle le câble (18, 118) et/ou le connecteur électrique (156) peuvent être reliés, et que le deuxième sas stérile (48, 148) présente une deuxième zone de liaison, à laquelle une zone de liaison complémentaire du premier sas stérile (42, 142) peut être reliée,
dans lequel la première zone de liaison (210) du deuxième sas stérile (48, 148) et la deuxième zone de liaison du deuxième sas stérile (48, 148) sont disposées de préférence sur des faces du deuxième sas stérile (48, 148) opposées l'une à l'autre.

9. Ensemble selon la revendication 8, **caractérisé en ce que** la première zone de liaison (210) du deuxième sas stérile (48, 148) peut être reliée au câble (18, 118) et/ou au connecteur électrique (156) prévu à l'extrémité du câble (18, 118) par l'intermédiaire d'une première liaison par encliquetage libérable, et que la deuxième zone de liaison du deuxième sas stérile (48, 148) peut être reliée à la zone de liaison complémentaire du premier sas stérile (42, 142) par l'intermédiaire d'une deuxième liaison par encliquetage libérable.

10. Ensemble selon l'une quelconque des revendications précédentes, **caractérisé en ce que** le premier clapet stérile (44, 46, 144, 146), lors de la liaison du premier sas stérile (42, 142) au deuxième sas stérile (48, 148), s'ouvre automatiquement et que le deuxième clapet stérile (50, 52, 150, 152), lors de la liaison du premier sas stérile (42, 142) au deuxième sas stérile (48, 148), s'ouvre automatiquement, que le premier clapet stérile (44, 46, 144, 146), lors de la séparation du premier sas stérile (42, 142) du deuxième sas stérile (48, 148), se ferme automatiquement et que le deuxième clapet stérile (50, 52, 150, 152), lors de la séparation du premier sas stérile (42, 142) du deuxième sas stérile (48, 148), se ferme automatiquement.

11. Ensemble selon la revendication 10, **caractérisé en ce que**, lors de la liaison du premier sas stérile (42, 142) au deuxième sas stérile (48, 148), un déverrouillage automatique du premier clapet stérile (44, 46, 144, 146) s'effectue,
**que**, lors de la liaison du premier sas stérile (42, 142) au deuxième sas stérile (48, 148), un déverrouillage automatique du deuxième clapet stérile (50, 52, 150, 152) s'effectue,
**que**, lors de la séparation du premier sas stérile (42, 142) du deuxième sas stérile (48, 148), un verrouillage automatique du premier clapet stérile (44, 46, 144, 146) s'effectue et
**que**, lors de la séparation du premier sas stérile (42, 142) du deuxième sas stérile (48, 148), un verrouillage automatique du deuxième clapet stérile (50, 52, 150, 152) s'effectue.

12. Ensemble selon l'une quelconque des revendications précédentes, **caractérisé en ce qu'**une face extérieure stérile du premier clapet stérile (44, 46, 144, 146), lors de la liaison du premier sas stérile (42, 142) au deuxième sas stérile (48, 148), est disposée à l'opposé sur la face extérieure stérile du deuxième clapet stérile (50, 52, 150, 152), lorsqu'aussi bien le premier clapet stérile (44, 46, 144, 146) que le deuxième clapet stérile (50, 52, 150, 152) sont ouverts, dans lequel la face extérieure du premier clapet stérile (44, 46, 144, 146) et la face extérieure du deuxième clapet stérile (50, 52, 150, 152) sont tournées l'une vers l'autre dans l'état ouvert.

13. Ensemble selon l'une quelconque des revendications précédentes, **caractérisé en ce qu'**une partie de l'enveloppe d'endoscope (16, 116) conçue pour loger et de préférence pour entourer une tige (126) d'un endoscope (14, 114) réalisé sous la forme d'un endoscope à barre, dans lequel l'enveloppe d'endoscope (12, 112) comprend un élément optique (30, 130), qui est disposé à l'opposé de l'extrémité de tige (28, 128) de l'endoscope (14, 114) lorsque l'endoscope (14, 114) est logé dans l'enveloppe d'endoscope (12, 112).ⁱ

14. Système pour la chirurgie robotisée, en particulier pour une procédure télérobotisée à l'intérieur d'une zone stérile (110),
avec au moins un ensemble (10, 100) selon l'une quelconque des revendications précédentes,
avec au moins un manipulateur (1200), qui présente un bras de manipulateur (1600a à 1600d),
avec au moins un dispositif d'entrée (4200) pour l'entrée d'au moins une commande d'entrée,
**caractérisé en ce que** l'endoscope (14, 114) logé dans l'enveloppe d'endoscope (12, 112) est relié au bras de manipulateur (1600a à 1600d),
**que** le système (1000) comprend une unité de commande (4000), qui positionne le bras de manipulateur (1600a à 1600d) et l'endoscope (14, 114) relié au bras de manipulateur (1600a à 1600d) en fonction de la commande d'entrée à l'aide d'au moins une unité d'entraînement.

15. Ensemble pour établir et libérer une liaison d'enfichage entre un premier connecteur électrique (138) non stérile et un deuxième connecteur électrique (156) non stérile complémentaire du premier connecteur électrique (138) dans un environnement stérile (110),
avec une première enveloppe stérile (112) destinée à loger au moins le premier connecteur électrique (138),
avec une deuxième enveloppe stérile (116) destinée à loger au moins le deuxième connecteur électrique (156),
**caractérisé en ce que**
la première enveloppe (112) comprend un premier sas stérile (142), qui présente au moins un premier clapet stérile (144, 146), qui, dans l'état fermé, protège le premier connecteur électrique (138) de manière stérile,
la deuxième enveloppe (116) comprend un deuxième sas stérile (148), qui présente au moins un deuxième clapet stérile (150, 152), qui, dans l'état fermé, protège le deuxième connecteur électrique (156) de manière stérile,
**que**, lors de la liaison du premier connecteur électrique (138) au deuxième connecteur électrique (156), un déplacement du premier clapet stérile (144, 146) de l'état fermé à un état ouvert et un déplacement du deuxième clapet stérile (150, 152) de l'état fermé à un état ouvert s'effectuent, de sorte qu'un couplage direct du premier connecteur électrique (138) au deuxième connecteur électrique (156) est possible, et
**que**, lors de la séparation du premier connecteur électrique (138) du deuxième connecteur électrique (156), un déplacement du premier clapet stérile (144, 146) de l'état ouvert à l'état fermé et un déplacement du deuxième clapet stérile (150, 152) de l'état ouvert à l'état fermé s'effectuent, de sorte que le premier clapet stérile (144, 146), après la séparation, protège de manière stérile au moins le premier connecteur électrique (138) et le deuxième clapet stérile (150, 152), après la séparation, protège de manière stérile le deuxième connecteur électrique (156).

16. Ensemble selon l'une quelconque des revendications précédentes, **caractérisé en ce que** le premier connecteur électrique (138) présente au moins un premier élément de contact électrique (174) destiné à établir une liaison électrique ou au moins un premier élément optique (172) destiné à établir une liaison optique,
**que** le deuxième connecteur électrique (156) présente au moins un deuxième élément de contact électrique (202) destiné à établir une liaison électrique avec le premier élément de contact électrique (174) ou au moins un deuxième élément optique (200) destiné à établir une liaison optique avec le premier élément optique (172),
**que** l'au moins un premier élément de contact électrique (174) et l'au moins un deuxième élément de contact électrique (202) et/ou que l'au moins un premier élément optique (172) et l'au moins un deuxième élément optique (200) sont disposés et réalisés de telle sorte que, lors d'une liaison entre le premier connecteur électrique (138) et le deuxième connecteur électrique (156), une liaison électrique entre le premier élément de contact électrique (174) et le deuxième élément de contact électrique (202) ou une liaison optique entre le premier élément optique (172) et le deuxième élément optique (200) s'effectue.

17. Ensemble selon l'une quelconque des revendications précédentes, **caractérisé en ce que** le premier connecteur électrique (138) et le deuxième connecteur électrique (156) sont réalisés de manière symétrique, de telle sorte que la liaison électrique entre le premier élément de contact électrique (174) et le deuxième élément de contact électrique (202) et/ou la liaison optique entre le premier élément optique (172) et le deuxième élément optique (200) sont possibles aussi bien lors de l'amenée des connecteurs électriques (138, 156) dans une première position et lors de l'amenée des connecteurs électriques (138, 156) dans une deuxième position, dans laquelle un des connecteurs électriques (138, 156) a été amené en rotation de 180° autour d'un axe s'étendant perpendiculairement au plan de contact des connecteurs électriques (138, 156).
